(19) Europäisches Patentamt
European Patent Office
Office européen des brevets

(11) EP 0 812 825 B1

(12) EUROPEAN PATENT SPECIFICATION

(45) Date of publication and mention of the grant of the patent: **11.02.2004 Bulletin 2004/07**

(21) Application number: **96940153.8**

(22) Date of filing: **29.11.1996**

(51) Int Cl.7: **C07D 209/12**, C07H 15/26, C07F 7/10, A61K 31/40, A61K 31/70

(86) International application number: **PCT/JP1996/003504**

(87) International publication number: **WO 1997/020814 (12.06.1997 Gazette 1997/25)**

# (54) PROPENONE DERIVATIVES

PROPENONDERIVATE

DERIVES DU PROPENONE

(84) Designated Contracting States: **AT BE CH DE DK ES FI FR GB GR IE IT LI LU MC NL PT SE**

(30) Priority: **01.12.1995 JP 31399895**

(43) Date of publication of application: **17.12.1997 Bulletin 1997/51**

(73) Proprietor: **KYOWA HAKKO KOGYO CO., LTD.**
**Chiyoda-ku, Tokyo 100 (JP)**

(72) Inventors:
- **IKEDA, Shun-ichi**
  **Machida-shi, Tokyo 194 (JP)**
- **KANAZAWA, Junji**
  **Sunto-gun, Shizuoka 411 (JP)**
- **SASAKI, Kimihito**
  **Sunto-gun, Shizuoka 411 (JP)**
- **NUKUI, Etsuko**
  **Sunto-gun, Shizuoka 411 (JP)**
- **OKABE, Masami**
  **Mishima-shi, Shizuoka 411 (JP)**
- **GOMI, Katsushige**
  **Susono-shi, Shizuoka 410-11 (JP)**
- **SAITO, Hiromitsu**
  **Kawasaki-shi, Kanagawa 214 (JP)**
- **SATO, Soichiro**
  **Mishima-shi, Shizuoka 411 (JP)**

(74) Representative: **VOSSIUS & PARTNER**
**Siebertstrasse 4**
**81675 München (DE)**

(56) References cited:
**WO-A-95/14003**
**JP-A- 8 277 242**
**JP-T- 5 507 072**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 812 825 B1

Printed by Jouve, 75001 PARIS (FR)

## Description

The present invention relates to propenone derivatives having an antitumor activity, an immunosuppressive activity, and a therapeutic effect for an autoimmune disease.

Typical examples of compounds having an antitumor activity include mitomycin C, adriamycin, vincristine, and the like, all of which are clinically useful as anticancer agents. However, since each of the compounds also has adverse effects such as myelotoxicity, cardiotoxicity, nerve damage, etc., a novel anticancer agent having less adverse effects is demanded. Further, an excellent immunosuppressive agent and a therapeutic agent for an autoimmune disease having reduced adverse effects are always demanded.

Propenone derivatives are known as having an anticancer activity (WO 95/19003). Chalcone derivatives are known as having the activity to inhibit polymerization of tubulin [Journal of the Medicinal Chemistry (J. Med. Chem.), 33, 1948 (1990) and Journal of Natural Products (J. Nat. Prod.), 56, 1718 (1993)]. Chalcone derivatives are also known as having an anticancer activity, being useful as a therapeutic agent for gout, and being useful as a therapeutic agent for multiple sclerosis (U. S. Patent Nos. 4904697, 4863968, and 4753965, respectively). 3-Indolyl-1-phenyl-2-propen-1-one derivatives are known to inhibit tyrosine phosphorylation of the receptor of a cellular proliferation factor [Cancer Research (Cancer Res.), 54, 6106 (1994) and WO 91/16305], to be useful as the organic nonlinear optical material (Japanese Published Unexamined Patent Application No. 255426/91), and to have the antiallergic activity [Khim.-Farm. Zh., 25, 18 (1991)].

Further, 3-(indol-3-yl)-1-phenyl-2-propen-1-one derivatives are disclosed in French Patent No. 2230349, Khim. Geterotsikl. Soedin, 1066 (1970), Khim. Geterotsikl. Soedin, 268 (1969), Khim, Geterotsikl. Soedin, 399 (1970), Farmaco Ed. Sci., 26, 591 (1971), etc.

The present invention relates to propenone derivatives represented by the following formula (I):

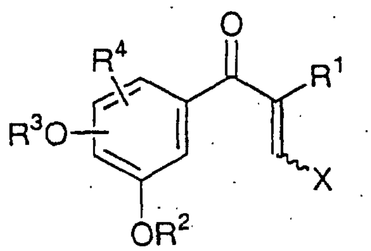

(I)

wherein

$R^1$ represents $C_1$-$C_6$ alkyl substituted with 1 to 4 hydroxy groups, or $YR^5$ (wherein Y represents S or O, and $R^5$ represents'substituted or unsubstituted $C_1$-$C_6$ alkyl, substituted or unsubstituted aryl; substituted or unsubstituted heteroaryl, or a substituted or unsubstituted cyclic ether residue), $R^2$ and $R^3$ represent independently hydrogen, $C_1$-$C_6$ alkyl, or substituted or unsubstituted aralkyl, or $R^2$ and $R^3$ are combined to represent substituted or unsubstituted methylene or ethylene, $R^4$, represents hydrogen, hydroxy, $C_1$-$C_6$ alkyl, substituted or unsubstituted aralkyl, $C_1$-$C_6$ alkoxy, substituted or unsubstituted aralkyloxy, or halogen, and X represents substituted or unsubstituted indolyl;

wherein the substituted $C_1$-$C_6$ alkyl has the same or different 1 to 4 substituents selected from the group consisting of vinyl, hydroxy, $C_1$-$C_6$ alkoxy, aryloxy, amino, $C_1$-$C_6$ alkylamino, di ($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl, tri($C_1$-$C_6$ alkyl)silyl and a cyclic ether residue; the substituted aryl, substituted heteroaryl, substituted cyclic ether residue, substituted aralkyl, and substituted aralkyloxy each has the same or different i to 4 substituents selected fromthe group consisting of $C_1$-$C_6$ alkyl, vinyl, hydroxy, hydroxymethyl, $C_1$-$C_6$ alkoxy, aryloxy, amino, $C_1$-$C_6$ alkylamino, di($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl, tri($C_1$-$C_6$ alkyl)silyl, aralkyl and a cyclic ether residue; the substituted methylene and substituted ethylene each has the same or different 1 to 3 $C_1$-$C_6$ alkyl;

the substituted indolyl has on the nitrogen atom at position 1 the substituent(s) selected from the group consisting of $C_1$-$C_6$ alkyl, $C_2$-$C_7$ alkanoyl, $C_1$-$C_6$ alkoxycarbonyl and aralkyl, and on the carbon atoms at positions 2 to 7 the substituent(s) selected from the group consisting of $C_1$-$C_6$ alkyl, $C_1$-$C_6$ alkoxy, amino, $C_1$-$C_6$ alkylamino, di ($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl and aralkyl; the aryl and the aryl moiety of the aryloxy represent phenyl or naphthyl;

the heteroaryl represents pyridyl, pyrazinyl, pyrimidinyl, pyridazinyl, quinolinyl, isoquinolinyl, phthalazinyl, quinazolinyl, quinoxalinyl, naphthyridinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl or purinyl;
the cyclic ether residue represents a cyclic ether residue having 2 to 6 carbon atoms; and
the aralkyl and the aralkyl moiety of the aralkyloxy represent an aralkyl group having 7 to 15 carbon atoms;

or a pharmaceutically acceptable salt thereof.

In the definitions of the groups of formula (I), the $C_1$-$C_6$ (lower) alkyl and the $C_1$-$C_6$ (lower) alkyl moiety of the $C_1$-$C_6$ (lower) alkoxy mean a straight-chain or branched alkyl group having 1 to 6 carbon atoms, such as methyl, ethyl, propyl, isopropyl, butyl, 2-butyl, isobutyl, tert-butyl, 1-pentyl, 2-pentyl, 3-pentyl, isoamyl, and hexyl. The aryl means phenyl or naphthyl, and the heteroaryl means pyridyl, pyrazinyl, pyrimidinyl, pyridazinyl, quinolinyl, isoquinolinyl, phthalazinyl, quinazolinyl, quinoxalinyl, naphthyridinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl or purinyl.

The cyclic ether residue means a cyclic ether residue having 2 to 6 carbon atoms, such as oxiranyl, oxetanyl, tetrahydrofuranyl, and tetrahydropyranyl. The aralkyl and the aralkyl moiety of the aralkyloxy mean an aralkyl group having 7 to 15 carbon atoms, such as benzyl, phenethyl, naphthylmethyl, and benzhydryl. The halogen includes fluorine, chorine, bromide, and iodine.

The substituted alkyl has the same or different 1 to 4 substituents selected from vinyl, hydroxy, lower alkoxy, aryloxy, amino, lower alkylamino, di(lower alkyl)amino, lower alkanoylamino, lower alkoxycarbonylamino, halogen, nitro, carboxy, lower alkanoyl, lower alkoxycarbonyl, tri(lower alkyl)silyl, and a cyclic ether residue. The substituted aryl, substituted heteroaryl, substituted cyclic ether residue, substituted aralkyl, and substituted aralkyloxy each has the same or different 1 to 4 substituents such as lower alkyl, vinyl, hydroxy, hydroxymethyl, lower alkoxy, aryloxy, amino, lower alkylamino, di(lower alkyl)amino, lower alkanoylamino, lower alkoxycarbonylamino, halogen, nitro, carboxy, lower alkanoyl, lower alkoxycarbonyl, tri(lower alkyl)silyl, aralkyl, and a cyclic ether residue. In the definitions of the substituents, the lower alkyl moiety of the lower alkylamino, di(lower alkyl)amino, lower alkanoylamino, lower alkoxycarbonylamino, and tri(lower alkyl)silyl has the same meaning as the lower alkyl defined above, and the aryl moiety of the aryloxy has the same meaning as the aryloxy defined above, and the lower alkyl, lower alkoxy, halogen, cyclic ether residue, and aralkyl each has the same meaning as defined above.

The substituted methylene and substituted ethylene each has the same or different 1 to 3 substituents of lower alkyl, and the lower alkyl has the same meaning as defined above.

Examples of the substituent on the nitrogen atom at position 1 of the substituted indolyl are lower alkyl, lower alkanoyl, lower alkoxycarbonyl, and aralkyl, and examples of the substituent on the carbon atoms at positions 2 to 7 of the substituted indolyl are lower alkyl, lower alkoxy, amino, lower alkylamino, di(lower alkyl)amino, lower alkanoylamino, lower alkoxycarbonylamino, halogen, nitro, carboxy, lower alkanoyl, lower alkoxycarbonyl, and aralkyl.

In the definitions of the substituents, the lower alkyl, lower alkoxy, lower alkylamino, di(lower alkyl)amino, lower alkanoylamino, lower alkoxycarbonylamino, halogen, lower alkanoyl, lower alkoxycarbonyl, and aralkyl each has the same meaning as defined above.

The pharmaceutically acceptable salts of Compounds (I) include inorganic acid addition salts such as hydrochloride, sulfate, and phosphate, organic acid addition salts such as acetate, maleate, fumarate, succinate, tartrate, citrate, oxalate, and methanesulfonate, alkali metal salts such as sodium salt and potassium salt, alkaline earth metal salts such as magnesium salt and calcium salt, metal salts such as aluminium salt and zinc salt, and ammonium salts such as ammonium and tetramethylammonium.

The present invention is described in detail below.

In the processes shown below, if the defined groups are converted into undesired groups under the conditions of the processes or are not suitable for carrying out the processes, the processes can be readily carried out by applying thereto means conventionally used in organic synthetic chemistry, for example, a means such as protection or deprotection of functional groups, or a method such as oxidation, reduction, or hydrolysis.

Compound (IV) is commercially available, is reported in a literature, or can be prepared according to the following reaction steps.

Process for Producing Compound (IV) - 1

Compound (IV) can be prepared according to the following reaction steps from the carboxylic acid or ester which is commercially available or known in the literature.

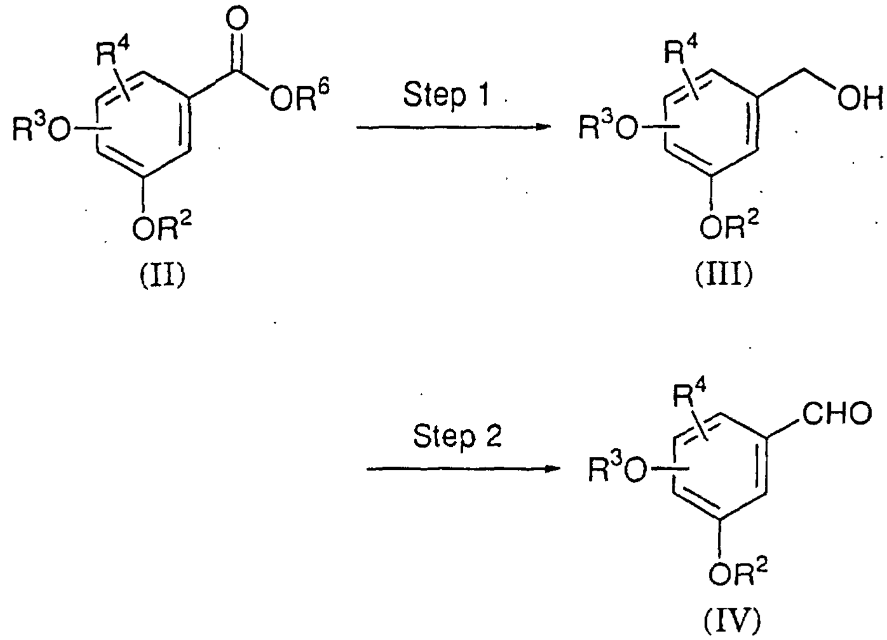


(II) (III) (IV)

(In the formulae, $R^6$ represents hydrogen, lower alkyl, or aralkyl; and $R^2$, $R^3$, and $R^4$ have the same meanings as defined above.)

The lower alkyl means a straight-chain or branched alkyl group having 1 to 6 carbon atoms and aralkyl has the same meaning as defined above.

Step 1

Compound (III) can be obtained by treating Compound (II) with a hydride reagent in an inert solvent. As the hydride reagent, 1 to 100 equivalents of sodium borohydride, lithium aluminum hydride, alane, borane, diisopropyl aluminium hydride, and the like may be used. As the solvent, aprotic solvents (for example, diethyl ether and tetrahydrofuran), aromatic hydrocarbons (for example, toluene), alcohols (for example, methanol and ethanol), and the like may be used alone or in combination. The reaction is carried out at the temperature between -78°C and the boiling point of the solvent employed in the reaction, and is completed in 0.1 hour to 3 days.

Step 2

Compound (IV) can be obtained by treating Compound (III) with an oxidizing agent in an inert solvent. As the oxidizing agent, 1 to 50 equivalents of chromium trioxide, a pyridine complex or hydrochloric acid complex thereof, potassium dichromate, manganese dioxide, 2,3-dichloro-5,6-dicyanobenzoquinone, and the like may be used. As the solvent, aprotic solvents (for example, acetone and N,N-dimethylformamide), halogenated hydrocarbons (for example, dichloromethane and chloroform), acetic acid, sulfuric acid, water, and the like may be used alone or in combination. The reaction is carried out at the temperature between -10°C and the boiling point of the solvent employed in the reaction, and is completed in 0.1 to 150 hours.

Process for Producing Compound (IV) - 2

Compound (IVb) which is Compound (IV) in which $R^2$ is lower alkyl or substituted or unsubstituted aralkyl can alternatively be prepared according to the following reaction step from the aldehyde (IVa) which is Compound (IV) in which $R^2$ is hydrogen, and commercially available or known in the literature.

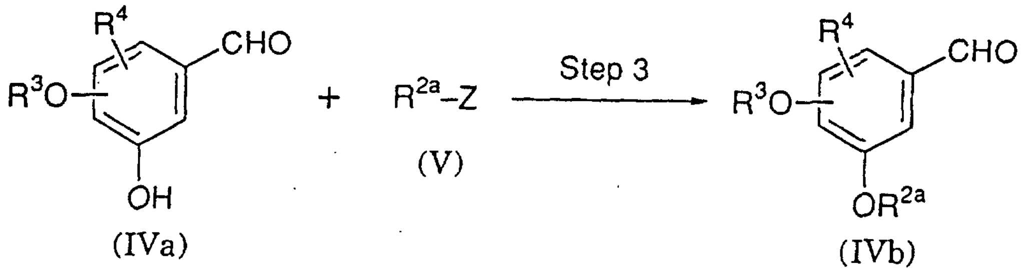

(IVa) (V) (IVb)

(In the formulae, $R^{2a}$ represents lower alkyl or substituted or unsubstituted aralkyl; Z represents halogen; and $R^3$ and $R^4$ have the same meanings as defined above.)

In the definition of $R^{2a}$, the lower alkyl and substituted or unsubstituted aralkyl have the same meanings as defined above. In the definition of Z, the halogen has the same meaning as defined above.

Step 3

Compound (IVb) can be obtained by reacting Compound (IVa) with Compound (V) in the presence of a base in an inert solvent. As the base, inorganic bases such as sodium hydroxide, potassium carbonate, sodium carbonate, and cesium fluoride, quaternary ammonium fluorides such as tetra-n-butylammonium fluoride, secondary amines such as piperidine, pyrrolidine, and morpholine, metal alkoxides such as sodium methoxide and potassium tert-butoxide, metal amides such as lithium diisopropylamide, metal hydrides such as sodium hydride, and the like may be used in an amount of 1 to 100 equivalents. As the solvent, aprotic solvents (for example, ethyl acetate, tetrahydrofuran, acetone, and N,N-dimethylformamide), aromatic hydrocarbons (for example, toluene), halogenated hydrocarbons (for example, chloroform), alcohols (for example, methanol and ethanol), and the like may be used alone or in combination. The reaction is carried out at the temperature between -78°C and the boiling point of the solvent employed in the reaction, and is completed in 0.1 hour to 3 days.

Process for Producing Compound (IV) - 3

Compound (IVd) which is Compound (IV) in which $R^3$ is lower alkyl or substituted or unsubstituted aralkyl can alternatively be prepared according to the following reaction step from the aldehyde (IVc) which is commercially available or known in the literature.

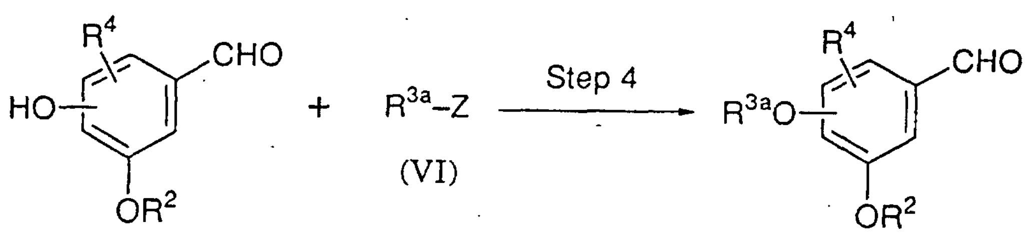

(IVc) (VI) (IVd)

(In the formulae, $R^{3a}$ represents lower alkyl or substituted or unsubstituted aralkyl; and $R^2$, $R^4$, and Z have the same meanings as defined above.)

Step 4

Compound (IVd) can be obtained by reacting Compound (IVc) with Compound (VI) according to the same method as that in Step 3.

Compound (IX) is commercially available, is reported in a literature, or can be prepared according to the following reaction steps.

Process for Producing Compound (IX) - 1

Compound (IX) can be prepared according to the following reaction steps.

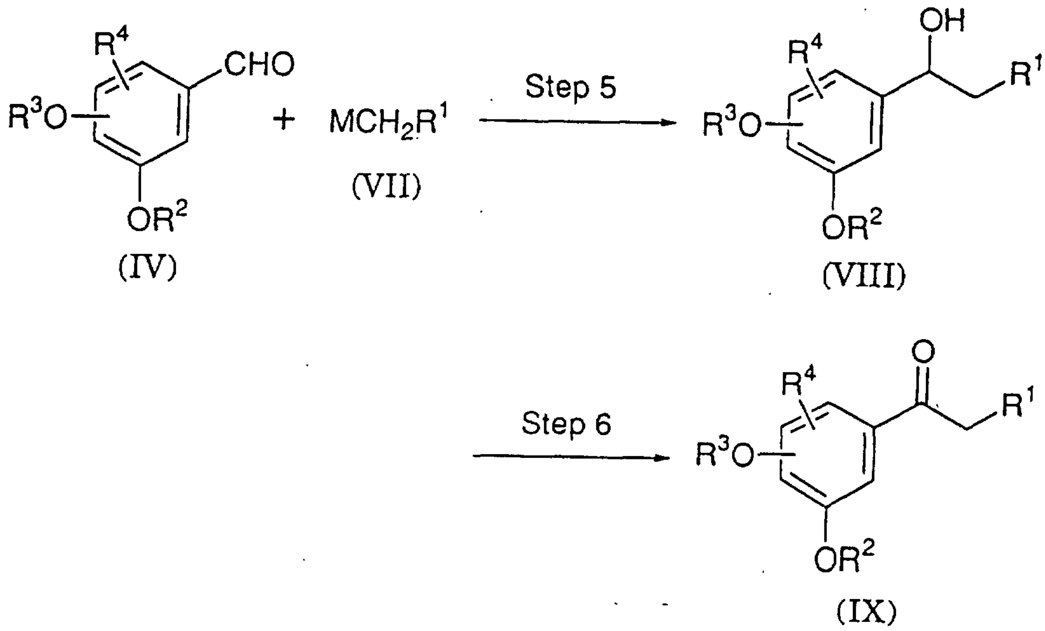

(IV) (VII) (VIII) (IX)

(In the formulae, M represents alkali metal, alkaline earth metal halide, or cerium dichloride; and $R^1$, $R^2$, $R^3$, and $R^4$ have the same meanings as defined above.)

In the definition of M, the alkali metal means lithium, sodium, potassium, cesium, or the like, and the alkaline earth metal halide means magnesium chloride, magnesium bromide, magnesium iodide, or the like.

Step 5

Compound (VIII) can be obtained by reacting Compound (IV) with 1 to 2 equivalents of Compound (VII) in an inert solvent. As the solvent, aprotic solvents (for example, diethyl ether and tetrahydrofuran), aromatic hydrocarbons (for example, toluene), and the like may be used alone or in combination. The reaction is carried out at the temperature between -100°C and the boiling point of the solvent employed in the reaction, and is completed in 0.1 to 24 hours.

Step 6

Compound (IX) can be obtained by treating Compound (VIII) according to the same method as that in Step 2.

Process for Producing Compound (IX) - 2

Compound (IXa) which is Compound (IX) in which $R^1$ is $YR^5$ can alternatively be prepared according to the following reaction steps.

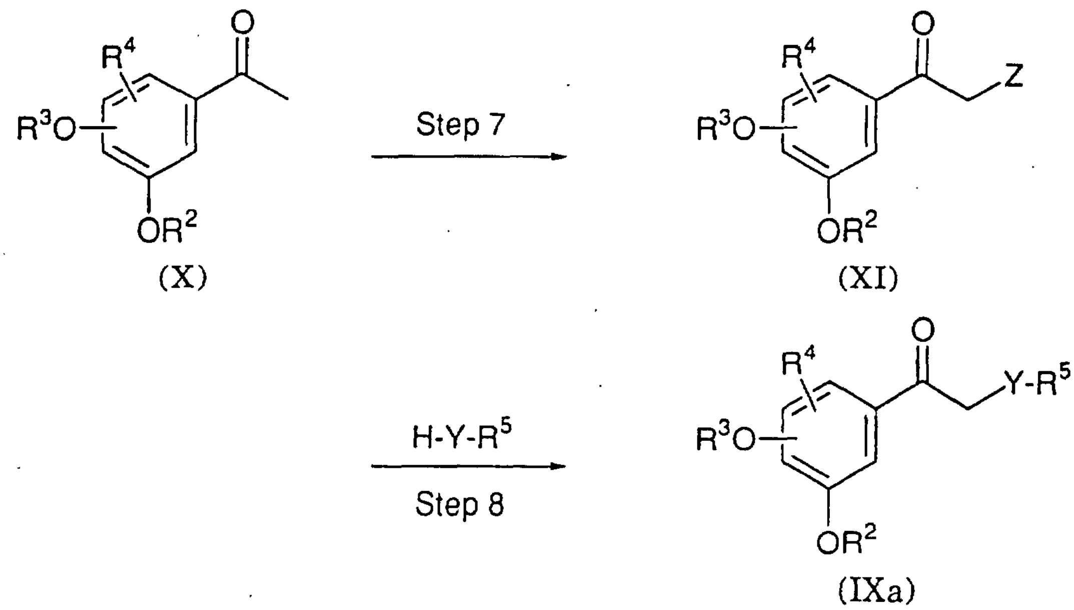


(In the formulae, $R^2$, $R^3$, $R^4$, $R^5$, Y, and Z have the same meanings as defined above.)

Step 7

Compound (XI) can be obtained by treating Compound (X) with a halogenating agent in an inert solvent. As the halogenating agent, 1 to 5 equivalents of pyrrolidone hydrotribromide, tetra-n-butylammonium tribromide, bromine, and the like may be used. As the solvent, aprotic solvents (for example, ethyl acetate and tetrahydrofuran), acetic acid, water, and the like may be used alone or in combination. The reaction is carried out at the temperature between 0°C and the boiling point of the solvent employed in the reaction, and is completed in 0.1 to 24 hours.

Step 8

Compound (IXa) can be obtained by reacting Compound (XI) with a compound represented by formula H-Y-$R^5$ in the presence of a base in an inert solvent. As the base, inorganic bases such as potassium carbonate, sodium carbonate, cesium fluoride, and sodium hydroxide, quaternary ammonium fluorides such as tetra-n-butylammonium fluoride, secondary amines such as piperidine, pyrrolidine, and morpholine, metal alkoxides such as sodium methoxide and potassium tert-butoxide, metal amides such as lithium diisopropylamide, metal hydrides such as sodium hydride, and the like may be used in an amount of 1 to 10 equivalents. As the solvent, aprotic solvents (for example, ethyl acetate, tetrahydrofuran, and dimethyl sulfoxide), aromatic hydrocarbons (for example, toluene), halogenated hydrocarbons (for example, chloroform), and the like may be used alone or in combination. The reaction is carried out at the temperature between -78°C and the boiling point of the solvent employed in the reaction, and is completed in 0.1 hour to one day.

Process for Producing Compound (IX) - 3

Compound (IX) can alternatively be prepared according to the following reaction step.

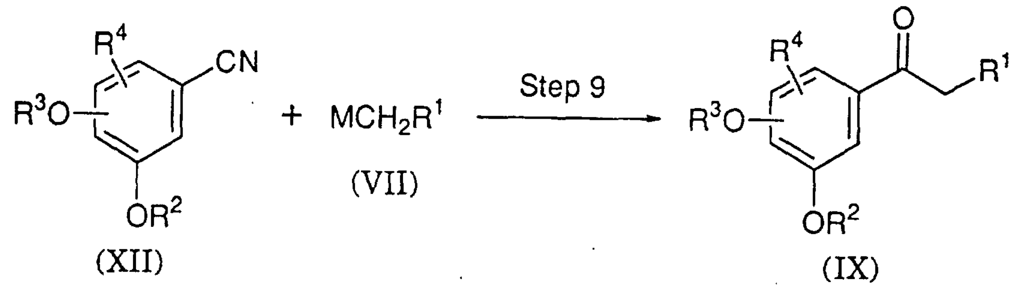


(XII) (VII) (IX)

(In the formulae, $R^1$, $R^2$, $R^3$, $R^4$, and M have the same meanings as defined above.)

Step 9

Compound (IX) can be obtained by reacting Compound (XII) with 1 to 2 equivalents of Compound (VII) in an inert solvent. As the solvent, aprotic solvents (for example, diethyl ether and tetrahydrofuran), aromatic hydrocarbons (for example, toluene), and the like may be used alone or in combination. The reaction is carried out at the temperature between 0°C and the boiling point of the solvent employed in the reaction, and is completed in 0.1 to 24 hours.

Process for Producing Compound (IX) - 4

Compound (IXc) which is Compound (IX) in which $R^2$ is lower alkyl or substituted or unsubstituted aralkyl can alternatively be prepared according to the following reaction step from Compound (IXb) which is Compound (IX) in which $R^2$ is hydrogen.

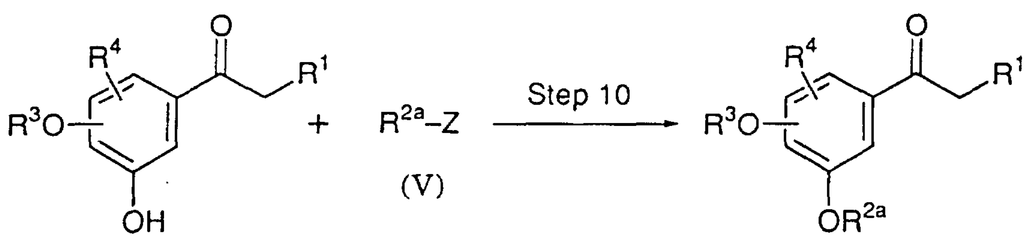


(IXb) (V) (IXc)

(In the formulae, $R^1$, $R^{2a}$, $R^3$, $R^4$, and Z have the same meanings as defined above.)

Step 10

Compound (IXc) can be obtained by reacting Compound (IXb) with Compound (V) according to the same method as that in Step 3.

Process for Producing Compound (IX) - 5

Compound (IXe) which is Compound (IX) in which $R^3$ is lower alkyl or substituted or unsubstituted aralkyl can alternatively be prepared according to the following reaction step from Compound (IXd) which is Compound (IX) in which $R^3$ is hydrogen.

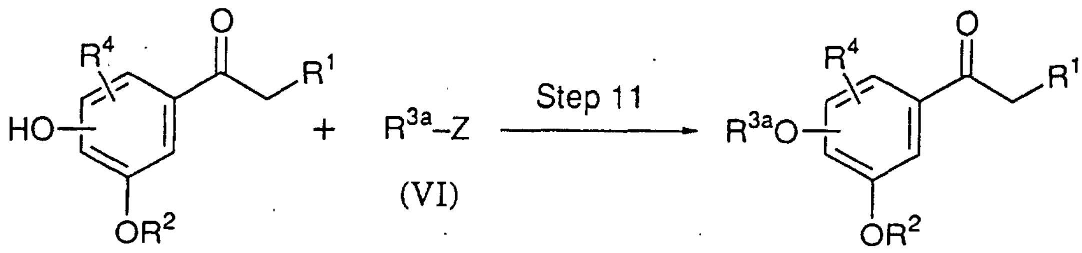


(IXd) (VI) (IXe)

(In the formulae, $R^1$, $R^2$, $R^{3a}$, $R^4$, and Z have the same meanings as defined above.)

Step 11

Compound (IXe) can be obtained by reacting Compound (IXd) with Compound (VI) according to the same method as that in Step 3.

Process for Producing Compound (IX) - 6

Compound (IXh) which is Compound (IX) having a 1,2-dihydroxyethyl group as a substituent of $R^1$ can alternatively be prepared according to the following reaction steps from Compound (IXf) having a vinyl group as a substituent of $R^1$.

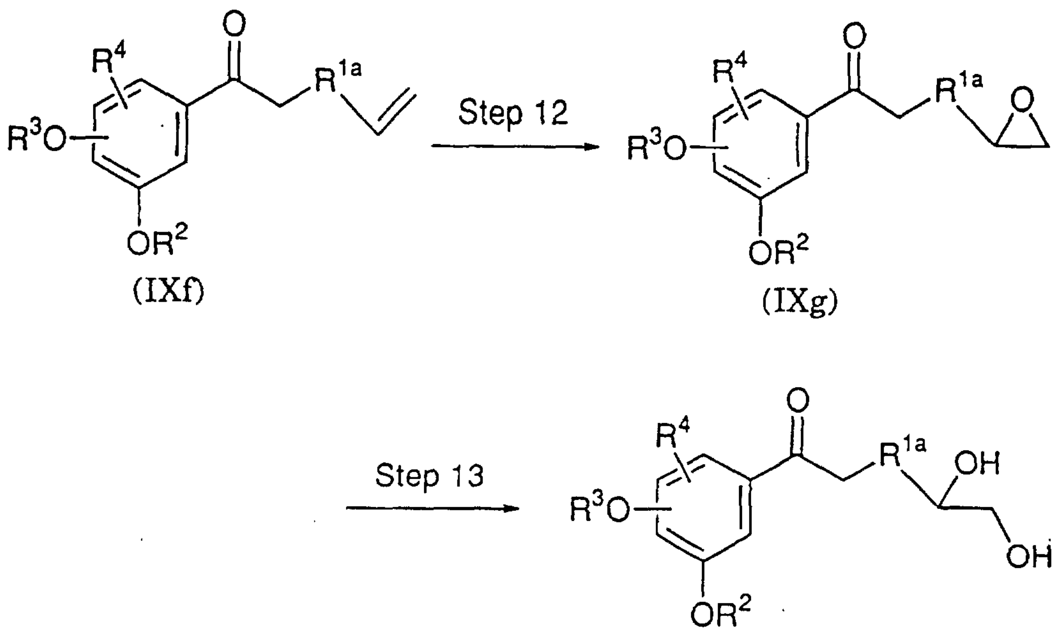


(IXf) (IXg) (IXh)

[In the formulae, $R^{1a}$ represents substituted lower alkyl or $YR^{5a}$ (wherein $R^{5a}$ represents substituted or unsubstituted lower alkyl, substituted or unsubstituted aryl, substituted or unsubstituted heteroaryl, or a substituted or unsubstituted cyclic ether residue; and Y has the same meaning as defined above); and $R^2$, $R^3$, and $R^4$ have the same meanings as defined above.]

In the definition of $R^{1a}$, the substituted lower alkyl has the same meaning as defined above. In the definition of $R^{5a}$, the substituted or unsubstituted lower alkyl, substituted or unsubstituted aryl, substituted or unsubstituted heteroaryl, and substituted or unsubstituted cyclic ether residue each has the same meaning as defined above.

Step 12

Compound (IXg) which is Compound (IX) having an oxiranyl group as a substituent of $R^1$ can be obtained by treating Compound (IXf) with an oxidizing agent, if necessary, in the presence of a catalyst or a base, in an inert solvent. As the oxidizing agent, 1 to 10 equivalents of m-chloroperbenzoic acid, tert-butyl hydroperoxide, hydrogen peroxide, and the like may be used. As the catalyst, 0.01 to 10 equivalents of vanadium acetylacetonate, titanium tetraisopropoxide, and the like may be used. As the base, 1 to 10 equivalents of sodium bicarbonate, potassium carbonate, sodium acetate, and the like may be used. As the solvent, aprotic solvents (for example, ethyl acetate and tetrahydrofuran), protic solvents (for example, methanol), halogenated hydrocarbons (for example, chloroform), acetic acid, water, and the like may be used alone or in combination. The reaction is carried out at the temperature between -30°C and the boiling point of the solvent employed in the reaction, and is completed in 0.1 to 72 hours.

Step 13

Compound (IXh) can be obtained by treating Compound (IXg) with a salt of a carboxylic acid or a base in an inert solvent. As the salt of a carboxylic acid, 1 to 100 equivalents of sodium acetate, potassium benzoate, and the like may be used. As the base, 1 to 100 equivalents of sodium bicarbonate, potassium carbonate, sodium acetate, and the like may be used. As the solvent, aprotic solvents (for example, N,N-dimethylformamide, pyridine, and tetrahydrofuran), protic solvents (for example, methanol), water, and the like may be used alone or in combination. The reaction is carried out at the temperature between 0°C and the boiling point of the solvent employed in the reaction, and is completed in 0.1 to 72 hours.

Process for Producing Compound (IX) - 7

Compound (IXa) can alternatively be prepared according to the following reaction steps from Compound (IV) or Compound (XI).

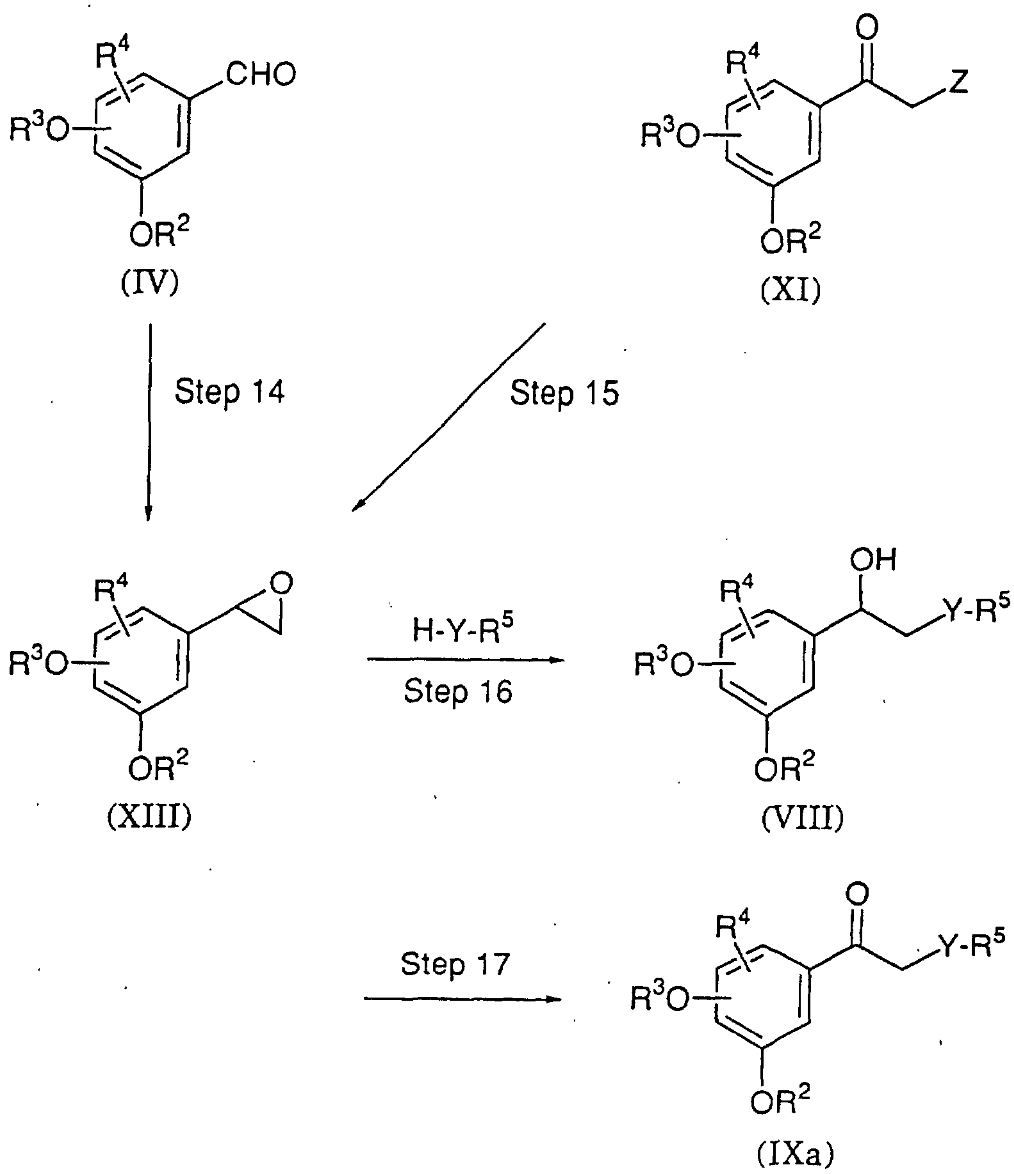


(IV) (XI) (XIII) (VIII) (IXa)

(In the formulae, $R^2$, $R^3$, $R^4$, $R^5$, Y, and Z have the same meanings as defined above.)

Step 14

Compound (XIII) can be obtained by reacting Compound (IV) with trimethylsulfoxonium iodide according to the same method as that in Step 8.

Step 15

Compound (XIII) can be obtained by treating Compound (XI) according to the same method as that in Step 1 or by adding a base thereafter and reacting the resulting mixture in an inert solvent at the temperature between 0°C and the boiling point of the solvent employed in the reaction for 1 to 24 hours. As the base, 1 to 10 equivalents of sodium bicarbonate, potassium carbonate, sodium acetate, and the like may be used. As the solvent, aprotic solvents (for example, diethyl ether and tetrahydrofuran), aromatic hydrocarbons (for example, toluene), alcohols (for example, methanol and ethanol), water, and the like may be used alone or in combination.

Step 16

Compound (VIII) can be obtained by reacting Compound (XIII) with Compound H-Y-$R^5$ according to the same

method as that in Step 8.

Step 17

Compound (IXa) can be obtained by treating Compound (VIII) according to the same method as that in Step 2.

Process for Producing Compound (I) - 1

Compound (I) can be prepared according to the following reaction step from Compound (IX) and Compound (XIV).

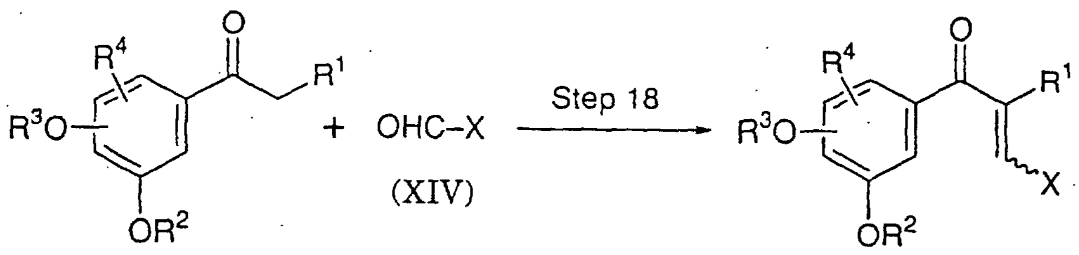


(XIV)

(IX) (I)

(In the formulae, $R^1$, $R^2$, $R^3$, $R^4$, and X have the same meanings as defined above.)

Step 18

Compound (I) can be obtained by reacting Compound (IX) with Compound (XIV) according to the same method as that in Step 3.

Process for Producing Compound (I) - 2

Compound (I) can alternatively be prepared according to the following reaction steps.

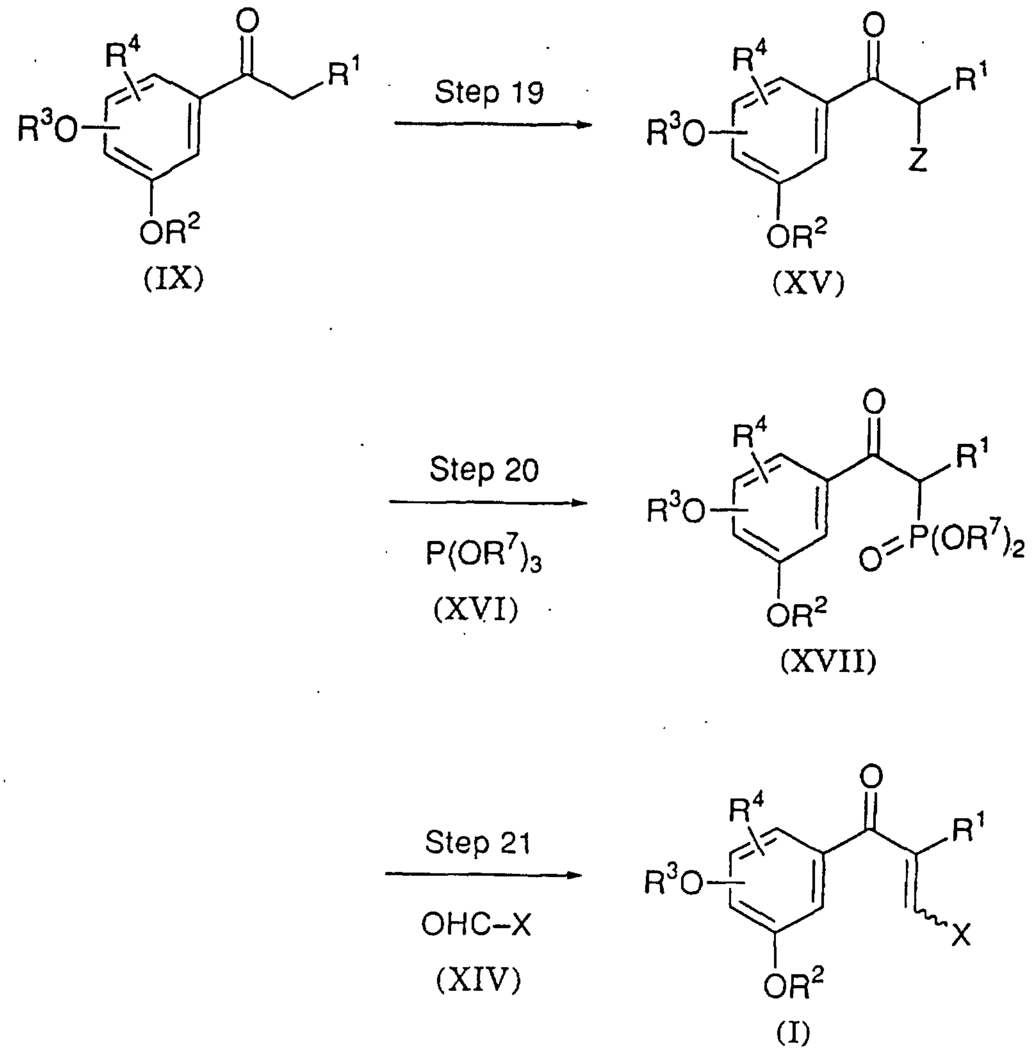


(In the formulae, $R^7$ represents lower alkyl; and $R^1$, $R^2$, $R^3$, $R^4$, X, and Z have the same meanings as defined above.)

In the definition of $R^7$, the lower alkyl has the same meaning as defined above.

Step 19

Compound (XV) can be obtained by treating Compound (IX) according to the same method as that in Step 7.

Step 20

Compound (XVII) can be obtained by reacting Compound (XV) with 1 to 10 equivalents of Compound (XVI) in an inert solvent or without a solvent. As the solvent, aprotic solvents (for example, ethyl acetate, tetrahydrofuran, toluene, benzene, and N,N-dimethylformamide), protic solvents (for example, methanol), and the like may be used alone or in combination. The reaction is carried out at the temperature between 0°C and 200°C, and is completed in 0.5 to 100 hours.

Step 21

Compound (I) can be obtained by reacting Compound (XVII) with Compound (XIV) according to the same method as that in Step 3.

Some Compounds (I) thus obtained can also be used as an intermediate to prepare novel derivatives (I) by subjecting them to oxidation, reduction, alkylation, acylation, or the like.

Compound (XIV) is commercially available, is reported in a literature (WO95/14003), or can be easily prepared

according to the reaction steps reported in a literature.

HS-$R^5$ which is the starting compound H-Y-$R^5$ in which Y is S is commercially available, is reported in a literature, or can be prepared according to the following reaction steps.

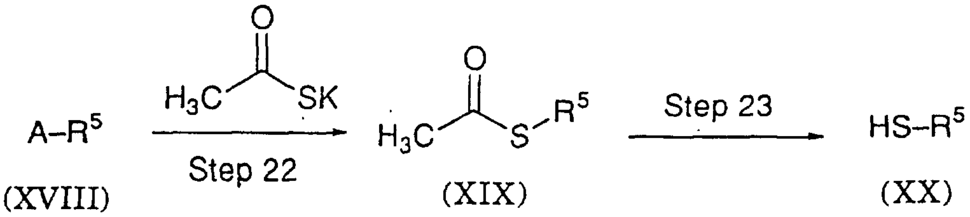


(XVIII) (XIX) (XX)

(In the formulae, A represents halogen, trifluoromethanesulfonyloxy, methanesulfonyloxy, benzenesulfonyloxy, or toluenesulfonyloxy; and $R^5$ has the same meaning as defined above.)

In the definition of A, the halogen has the same meaning as defined above.

Step 22

Compound (XIX) can be obtained by reacting Compound (XVIII) with 1 to 50 equivalents of potassium thioacetate in an inert solvent. As the solvent, aprotic solvents (for example, N,N-dimethylformamide, N-methylpyrrolidone, dimethyl sulfoxide, pyridine, and tetrahydrofuran), protic solvents (for example, methanol), water, and the like may be used alone or in combination. The reaction is carried out at the temperature between 0°C and the boiling point of the solvent employed in the reaction, and is completed in 0.1 to 72 hours.

Step 23

Compound (XX) can be obtained by treating Compound (XIX) with a base in an inert solvent or without a solvent. As the base, inorganic bases such as sodium bicarbonate, potassium carbonate, sodium acetate, and sodium hydroxide, amines such as piperidine, pyrrolidine, and morpholine, and the like may be used in an amount of 1 to 100 equivalents. As the solvent, aprotic solvents (for example, N,N-dimethylformamide, pyridine, and tetrahydrofuran), protic solvents (for example, methanol), water, and the like may be used alone or in combination. The reaction is carried out at the temperature between 0°C and the boiling point of the solvent employed in the reaction, and is completed in 0.1 to 72 hours.

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without isolation.

In the case where a salt of Compound (I) is desired and it is produced in the form of the desired salt, it can be subjected to purification as such. In the case where Compound (I) is produced in the free form and its salt is desired, Compound (I) is dissolved or suspended in a suitable organic solvent, followed by addition of an acid or a base to form a salt by a conventional method.

Compounds (I) can exist in the form of E/Z geometrical isomers, and the present invention covers all isomers including these geometrical isomers and mixtures thereof. In the case where Compound (I) is obtained in a E/Z mixture, and separation of E/Z isomers is desired, they can be isolated and purified by fractionation methods, for example, fractional crystallization, fractional precipitation, fractional dissolution, or the like.

Compounds (I) and pharmaceutically acceptable salts thereof may be in the form of adducts with water or various solvents, which are also within the scope of the present invention.

Examples of Compounds (I) obtained in the processes described above are shown in Table 1.

Table 1-1

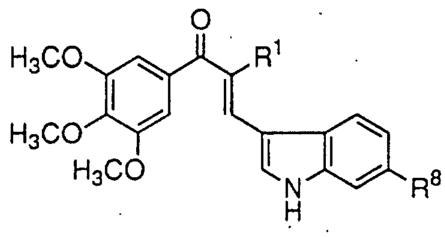

| Compd. No. | $R^1$ | $R^8$ |
|---|---|---|
| 1 | $OCH_3$ | H |
| 2 | $OCH_2CH_3$ | H |
| 3 | $O(CH_2)_2CH_3$ | H |
| 4 | $OCH(CH_3)_2$ | H |
| 5 | $O(CH_2)_2Si(CH_3)_3$ | H |
| 6 | $O(CH_2)_3Si(CH_3)_3$ | H |
| 7 | 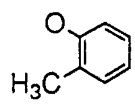 | H |
| 8 | 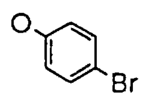 | H |
| 9 | $SCH_3$ | H |
| 10 | $S(CH_2)_2Si(CH_3)_3$ | H |
| 11 | 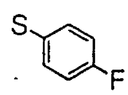 | H |
| 12 | $S(CH_2)_2OH$ | H |
| 13 | $S(CH_2)_2OH$ | $CH_3$ |
| 14 | 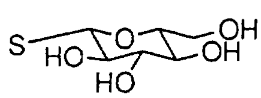 | $CH_3$ |
| 15 | $SCH_2CO_2H$ | H |
| 16 | $SCH_2CO_2CH_3$ | H |
| 17 | $S(CH_2)_2N(CH_2CH_3)_2$ | H |
| 18 | $S(CH_2)_4OC_6H_5$ | H |
| 19 | $(CH_2)_2CH(OH)CH_2OH$ | H |
| 20 | $(CH_2)_2CH(OH)CH_2OH$ | $CH_3$ |

Table 1-2

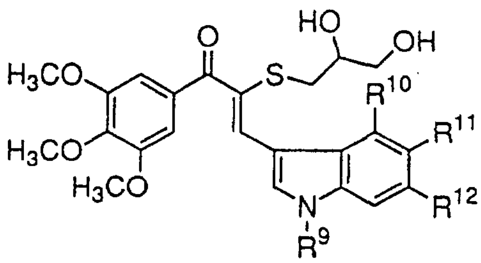

| Compd. No. | $R^9$ | $R^{10}$ | $R^{11}$ | $R^{12}$ |
|---|---|---|---|---|
| 21 | H | H | H | H |
| 22 | $CH_3$ | H | H | H |
| 23 | H | $CH_3$ | H | H |
| 24 | H | Cl | H | H |
| 25 | H | H | $CH_3$ | H |
| 26 | H | H | Cl | H |
| 27 | H | H | F | H |
| 28 | H | H | H | $CH_3$ |
| 29 | H | H | H | $CH_2CH_3$ |
| 30 | H | H | H | $CH(CH_3)_2$ |
| 31 | H | H | H | Cl |
| 32 | H | H | H | F |
| 33 | H | H | H | $NHCOCH_3$ |
| 34 | $CH_3$ | H | H | $NHCOCH_3$ |

Table 1-3

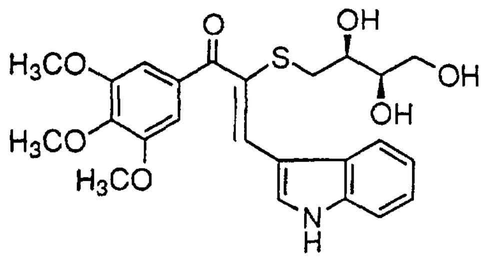

Compound 35

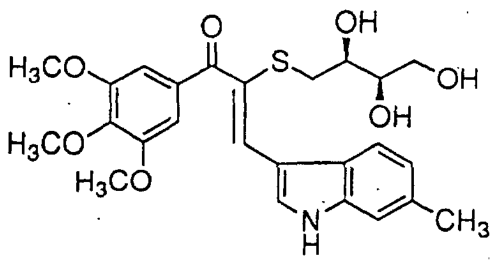

Compound 36

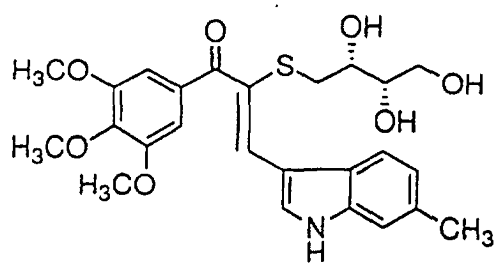

Compound 37

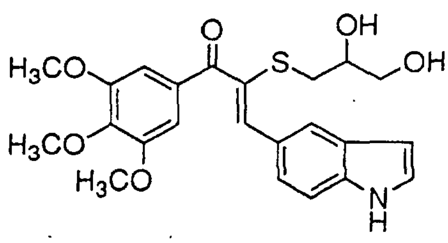

Compound 38a

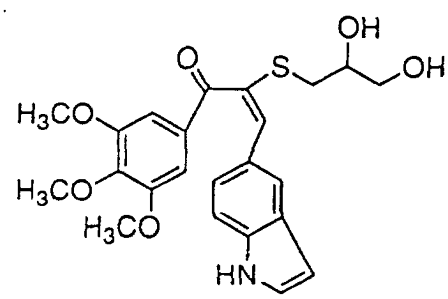

Compound 38b

The pharmacological activities of Compounds (I) are shown in detail below by test examples.

Test Example 1: HeLa $S_3$ Cell Growth Inhibition Test

Each 0.1 ml of HeLa $S_3$ cells which had been prepared to 3 x $10^4$ cells/ml using a medium consisting of MEM medium, 10% fetal bovine serum, and 2 mM glutamine was distributed in each well of 96 well-microtiter plate.

HeLa $S_3$ was cultured at 37°C for one night in a $CO_2$ incubator, each 0.05 ml of test compounds which had been appropriately diluted with the culture solution was added thereto, and the mixture was cultured at 37°C for 72 hours in a $CO_2$ incubator. Supernatant was removed, each 0.1 ml of the culture solution containing 0.02% neutral red was added to the residue, the mixture was incubated at 37°C for one hour in a $CO_2$ incubator, and the cells were stained. Supernatant was removed and the residue was washed once with physiological saline. Then, the pigment was extracted with 0.001 N hydrochloric acid/30% ethanol and the absorbance at 550 nm was measured by a microplatereader. A concentration of the test compound ($IC_{50}$) at which the growth of cell is inhibited by 50% was calculated by comparing the absorbance of non-treated cells and that of cells treated with a predetermined concentration of the

test compound.

The results are shown in Table 2.

Table 2

| Compd. No. | $IC_{50}$ (72 hrs, nM) |
|---|---|
| 1 | 2.4 |
| 2 | 2.5 |
| 3 | 13 |
| 4 | 3.7 |
| 7 | 68 |
| 8 | 62 |
| 9 | 2.2 |
| 13 | 2.5 |
| 16 | 4.7 |
| 19 | 2.1 |
| 20 | 5.6 |
| 21 | 4.8 |
| 22 | 20 |
| 23 | 19 |
| 24 | 15 |
| 27 | 6.3 |
| 28 | 6.3 |
| 29 | 18 |
| 31 | 7.2 |
| 32 | 5.9 |
| 35 | 4.4 |
| 36 | 5.3 |
| 37 | 7.1 |

Test Example 2: Effect upon P388 Ascites Tumor

The experiment was carried out by using groups of 6-weeks-old male $CDF_1$ mice, each group consisting of five mice. $10^6$ cells of P388 mouse leukemia were implanted into the abdominal cavities of the mice. A test compound was sufficiently wetted by adding 10 μl of Tween 80 relative to 1 mg of a sample, and 0.3 % CMC (sodium carboxymethyl cellulose) solution was then added to the test compound to form a suspension. The resultant suspension was administered once 24 hours after implantation of the tumor, or repeatedly for consecutive 5 days from 24 hours after implantation of the tumor. The average survival day (T) in a group was calculated from the survival days of the respective mice in the group administered with the test compound at each dose. On the other hand, the average survival day (C) of a group which was not administered was measured, and the increased life span [ILS (%)] was calculated according to the following equation:

$$[(T - C)/C] \times 100\ (\%)$$

The results are shown in Table 3.

Table 3

| Compd. No. | ILS (%) [Dose (mg/kg)] | |
|---|---|---|
| | five consec. admin. | single admin. |
| 1 | 50 ( 5.0) | 67 ( 25) |
| 2 | 35 ( 10) | 34 ( 50) |
| 3 | 41 ( 13) | 25 ( 50) |
| 8 | 31 ( 50) | 49 (200) |

Table 3 (continued)

| Compd. No. | ILS (%) [Dose (mg/kg)] | |
|---|---|---|
| | five consec. admin. | single admin. |
| 9 | 33 ( 5.0) | 18 ( 13) |
| 10 | 73 (100) | 16 (100) |
| 11 | 47 ( 25) | 38 ( 50) |
| 12 | 52 ( 20) | 20 ( 50) |
| 13 | 67 ( 5.0) | 40 ( 25) |
| 21 | 74 ( 10) | 48 ( 50) |
| 27 | 99 ( 20) | 65 ( 50) |
| 28 | 54 ( 10) | 24 ( 13) |
| 31 | 70 ( 20) | 52 ( 50) |

Test Example 3: Effect against Delayed Type Hypersensitivity Footpad Reaction

Male BALB/c mice (8-weeks-old, Charles River Japan Inc.) were immunized by intradermally injection in the dorsal flank of 100 µl of 10mM 2,4,6-trinitrobenzenesulfonic acid (TNBS) in saline. Five mice were used in each group, that is, control group [0.5% methylcellulose containing 10% dimethyl sulfoxide (DMSO)-administered group], Cyclosporine A (Sandoz)-administered group, and test compound-administered group wherein a determined concentration of test compound suspended in 0.5% methylcellulose containing 10% DMSO was administered. Each of Cyclosporine A and test compound was intraperitoneally administered 30 minutes before and once a day for 4 days after immunization. 50 µl of 10mM TNBS was intradermally injected as a challenging antigen 5 days after immunization when antigen-sensitization was developed. Thickness of both footpads of each animal in each dose of test compound-administered group was measured with a dial thickness gauge 18 hours after antigen challenge, and the value of the difference in thickness of left and right footpad (T) was determined. On the other hand, thickness of both footpads in control group was measured, and the value of the difference in thickness of left and right footpad (C) was determined. Suppression rate in footpad reaction was calculated as [(C - T)/C] x 100. The results are shown in Table 4.

Table 4

| Compd. No. | Suppression rate(%) [Dose (mg/kg x 5)] |
|---|---|
| 21 | 8.5 (3.0) |
| | 69 (10) |
| Cyclosporine A | 89 (30) |

Test Example 4: Effect against anti-Trinitrophenol (TNP) Antibody Production

Mice which were used for the effect against delayed type hypersensitivity footpad reaction were bled, the sera were separated by centrifugation at 3000rpm at 4°C for 10 minutes, and then anti-TNP antibody titer was measured. For measuring anti-TNP antibody, enzyme-linked immunosorbent assay (ELISA) was used. 96 Well microtiter plates for ELISA (NUNC Inc.) were coated with TNP-BSA which was prepared according to the method of Schmitt-Verhulst, et al. [Journal of Experimental Medicine, 147, 352 (1978)], and after coating, culture supernatant was added and the mixture was reacted. Peroxidase labeled anti-mouse IgG+IgA+IgM (American Qualex International, Inc.) was added and bound to anti-TNP antibody in culture supernatant which was bound to plate. Orthophenylenediamine (Wako Pure Chemical Industries, Ltd) solution containing hydrogen peroxide (Wako Pure Chemical Industries, Ltd) was added and enzyme reaction was started. After sufficient coloring, coloring reaction was stopped by addition of 10% sulfonic acid (Wako Pure Chemical Industries, Ltd) and absorbance at 490nm was measured by immunoreader (Intermed Japan, NJ-2000).

The results are shown in Table 5. Suppression rate of anti-TNP antibody production by test compound was calculated according to the following equation.

$$\text{Suppression rate (\%)} = \frac{\text{(Absorbance in control group) - (Absorbance in test group)}}{\text{Absorbance in control group}} \times 100$$

Table 5

| Compd. No. | Suppression rate(%) [Dose (mg/kg x 5)] |
|---|---|
| 21 | 15 (3.0) |
| | 57 (10) |
| Cyclosporine A | 39 (30) |

Test Example 5: T-Cell Proliferation Inhibitory Test Using Mouse Mixed Lymphocyte Reaction (MLR)

Spleen was sterilly removed from C3H/He mouse and single cell suspension was prepared. This suspension was irradiated with 2000R X-ray and adjusted into 8 x $10^6$ cells/ml. 50 μl of lymph-node cells of BALB/c mouse (containing 2 x $10^5$ cells), 50 μl of X-ray irradiated spleen cell suspension of C3H/He mouse (containing 4 x $10^5$ cells), and 50 μl of determined concentration of test compound solution were added in each well of 96 well microtiter plate and cultured at 37°C for 72 hours in the $CO_2$ incubator. Eight hours before the end of culture, [$^3$H]-Thymidine (1.85 KBq) was added. At the end of culture, cells were trapped. on the filter paper by cell harvester and dried. Toluene type scintillator was added and radioactivity of [$^3$H]-Thymidine incorporated into cells was measured by liquid scintillation counter.

The results are shown in Table 6. Suppression rate of T-cell proliferation was calculated according to the following equation.

$$\text{Suppression rate (\%)} = \frac{\text{(Radioactivity in control group) - (Radioactivity in test group)}}{\text{(Radioactivity in control group) - (Radioactivity in X-ray irradiated C3H/He+Radioactivity in Balb/c)}} \times 100$$

Table 6

| Compd. No. | Suppression rate(%) [Concentration (M)] |
|---|---|
| 21 | 107 ($10^{-7}$)<br>106 ($10^{-8}$)<br>7. 8 ($10^{-9}$) |

The compounds obtained according to the present invention are useful as antitumor agents, immunosuppresive agent, and therapeutic agents for an autoimmune disease, and can be used as they are or in various administration forms. For example, when Compounds (I) are used as injections, Compounds (I) may be dissolved in a diluting agent conventionally used in this field such as physiological saline, glucose injections, lactose injections, mannitol injections, or the like, freeze-dried on the basis of the Japanese Pharmacopoeia, or mixed with sodium chloride to form powder injections. These injections may contain an adjuvant such as polyethylene glycol, HCO-60 (surfactant: produced by Nikko Chemical Co., Ltd.), or the like; and a carrier such as ethanol and/or liposome, cyclodextrin, or the like. Although the injections are generally subjected to intravenous administration, they can also be subjected to arterial administration, intra-abdominal administration, or intrathoracic administration.

When Compounds (I) are mixed with appropriate excipients, disintegrators, binders, lubricants, and the like and formed to tablets, granules, powders, syrups, or the like by a conventional method, the compounds can also be used as oral agents. Compounds (I) may be mixed with carriers conventionally used and formed, by a conventional method, to suppositories which can be administered to the rectum.

The dose varies depending upon the mode of administration, the type of Compound (I), the age and conditions of a patient, etc., and the administration schedule can be changed according to the conditions of a patient or the dose. For example, administration can be made at a dose of 0.01 to 100 mg/kg once a week or once every three weeks.

Reference Examples and Examples are described below.

Best Mode for Carrying Out the Invention

The physicochemical data of each compound were measured by the following apparatus.

$^1$H-NMR: Nihon Denshi JNM-GX270 (270 MHz) Hitachi R-90H (90 MHz)
MS: Nihon Denshi JSM-D300
Elemental Analysis: Perkin Elmer 2400 CHN Analyzer

Reference Example 1

2-Bromo-3',4',5'-trimethoxyacetophenone (Compound XIa)

3',4',5'-Trimethoxyacetophenone (15.0 g) and pyrrolidone hydrotribromide (35.39 g) were dissolved in tetrahydrofuran (225 ml), followed by stirring at 40°C for one hour. The reaction solution was cooled to room temperature, precipitated crystals were filtered off, and the filtrate was concentrated under reduced pressure. The residue was purified by silica gel column chromatography, and the obtained crude crystals were washed with hexane (100 ml) to give 2-bromo-3',4',5'-trimethoxyacetophenone (Compound XIa, 12.20 g).

$^1$H-NMR (90 MHz, $CDCl_3$) δ 3.92 (s, 6H), 3.94 (s, 3H), 4.41 (s, 2H), 7.24 (s, 2H)

EI-MS m/z = 288, 290 ($M^+$)

| Elemental Analysis: $C_{11}H_{13}BrO_4$ | | |
|---|---|---|
| Calcd.(%): | C, 45.70; | H, 4.53 |
| Found (%): | C, 45.61; | H, 4.30 |

Reference Example 2

3',4',5'-Trimethoxystyrene oxide (Compound XIIIa)

2-Bromo-3',4',5'-trimethoxyacetophenone (Compound XIa, 10.00 g) obtained in Reference Example 1 was dissolved in methanol (350 ml), and sodium borohydride (1.31 g) was added thereto, followed by stirring at room temperature for 1.5 hours. Sodium borohydride (1.38 g) was added to the reaction solution and the mixture was stirred at room temperature for 0.5 hours. The reaction solution was concentrated under reduced pressure and the residue was purified by silica gel column chromatography to give 3',4',5'-trimethoxystyrene oxide (Compound XIIIa, 4.95g).

$^1$H-NMR (90 MHz, $CDCl_3$) δ 2.74 (dd, J = 5.5, 2.4 Hz, 1H), 3.14 (dd, J = 5.5, 4.3 Hz, 1H), 3.83 (m, 1H), 3.86 (s, 9H), 6.51 (s, 2H)

EI-MS m/z = 210 ($M^+$)

Reference Example 3

3',4',5'-Trimethoxystyrene oxide (Compound XIIIa)

3,4,5-Trimethoxybenzaldehyde (1.84 g) and trimethylsulfoxonium iodide (2.28 g) were dissolved in dimethyl sulfoxide (18 ml), and sodium hydride (414.4 mg). was added thereto, followed by stirring at room temperature for one hour. The reaction solution was subjected to partitioning between ethyl acetate and water, and the organic layer was successively washed with water and a saturated saline and dried over anhydrous sodium sulfate. The organic solvent was evaporated under reduced pressure to give 3',4',5'-trimethoxystyrene oxide (Compound XIIIa, 1.30 g).

Reference Example 4

2-Methoxy-3',4',5'-trimethoxyacetophenone (Compound IX-1)

3',4',5'-Trimethoxystyrene oxide (Compound XIIIa, 700.0 mg) obtained in Reference Example 2 was dissolved in methanol (100 ml), and sodium methoxide (0.90 g) was added thereto, followed by heating under reflux for 6 hours. The reaction solution was subjected to partitioning between chloroform and water, and the organic layer was washed with water, dried over anhydrous magnesium sulfate, and concentrated under reduced pressure. The residue was dissolved in acetone (29 ml), and Jones' reagent (1.5 ml) was added thereto under ice-cooling, followed by stirring for 30 minutes. 2-Propanol (1.5 ml) was added to the reaction solution, and the solution was concentrated under reduced pressure, followed by partitioning between ethyl acetate and water. The organic layer was successively washed with water and a saturated saline, dried over magnesium sulfate, and concentrated under reduced pressure. The residue was purified by silica gel column chromatography to give 2-methoxy-3',4',5'-trimethoxyacetophenone (Compound IX-1, 363.2 mg).

$^1$H-NMR (90 MHz, $CDCl_3$) δ 3.51 (s, 3H), 3.92 (s, 9H), 4.65 (s, 2H), 7.21 (s, 2H)

EI-MS m/z = 240 ($M^+$)

Reference Example 5

2-Ethoxy-3',4',5'-trimethoxyacetophenone (Compound IX-2)

3',4',5'-Trimethoxystyrene oxide (Compound XIIIa, 1.00 g) obtained in Reference Example 2 was dissolved in ethanol (14 ml), and potassium tert-butoxide (1.07 g) was added thereto, followed by heating under reflux for one hour. The reaction solution was subjected to partitioning between ethyl acetate and water, and the organic layer was washed with water, dried over anhydrous magnesium sulfate, and concentrated under reduced pressure. The residue was dissolved in acetone (33 ml), and Jones' reagent (1.7 ml) was added thereto under ice-cooling, followed by stirring for 30 minutes. 2-Propanol (1.7 ml) was added to the reaction solution, and the solution was concentrated under reduced pressure, followed by partitioning between ethyl acetate and water. The organic layer was successively washed with water and a saturated saline, dried over magnesium sulfate, and concentrated under reduced pressure. The residue was purified by silica gel column chromatography to give 2-ethoxy-3',4',5'-trimethoxyacetophenone (Compound IX-2, 615.0 mg).

$^1$H-NMR (90 MHz, $CDCl_3$) $\delta$ 1.27 (t, J = 7.0 Hz, 3H), 3.68 (q, J = 7.0 Hz, 2H), 3.91 (s, 9H), 4.64 (s, 2H), 7.28 (s, 2H)

EI-MS m/z = 254 ($M^+$)

Reference Example 6

3',4',5'-Trimethoxy-2-propyloxyacetophenone (Compound IX-3)

Substantially the same procedure as in Reference Example 5 was repeated using 3',4',5'-trimethoxystyrene oxide (Compound XIIIa, 1.00 g) obtained in Reference Example 2 and 1-propanol (17.8 ml) to give 3',4',5'-trimethoxy-2-propyloxyacetophenone (Compound IX-3, 615.0 mg).

$^1$H-NMR (90 MHz, $CDCl_3$) $\delta$ 0.95 (t, J = 6.9 Hz, 3H), 1.64 (m, 2H), 3.53 (q, J = 6.9 Hz, 2H), 3.91 (s, 9H), 4.65 (s, 2H), 7.26 (s, 2H)

FAB-MS m/z = 269 ($M^+$+1)

Reference Example 7

2-Isopropyloxy-3',4',5'-trimethoxyacetophenone (Compound IX-4)

Substantially the same procedure as in Reference Example 5 was repeated using 3',4',5'-trimethoxystyrene oxide (Compound XIIIa, 1.00 g) obtained in Reference Example 2 and 2-propanol (18.2 ml) to give 2-isopropyloxy-3',4',5'-trimethoxyacetophenone (Compound IX-4, 548.9 mg).

$^1$H-NMR (90 MHz, $CDCl_3$) $\delta$ 1.24 (d, J = 7.0 Hz, 6H), 3.68 (m, 1H), 3.91 (s, 9H), 4.67 (s, 2H), 7.25 (s, 2H)

EI-MS m/z = 268 ($M^+$)

Reference Example 8

3',4',5'-Trimethoxy-2-(2-trimethylsilylethoxy)acetophenone (Compound IX-5)

Substantially the same procedure as in Reference Example 5 was repeated using 3',4',5'-trimethoxystyrene oxide (Compound XIIIa, 1.05 g) obtained in Reference Example 2 and 2-trimethylsilylethanol (35.8 ml) to give 3',4',5'-trimethoxy-2-(2-trimethylsilylethoxy)acetophenone (Compound IX-5, 825.0 mg).

$^1$H-NMR (270 MHz, $CDCl_3$) $\delta$ 0.02 (s, 9H), 1.04 (t, J = 8.5 Hz, 2H), 3.66 (t, J = 8.5 Hz, 2H), 3.91 (s, 6H), 3.92 (s, 3H), 4.67 (s, 2H), 7.24 (s, 2H)

EI-MS m/z = 326 ($M^+$)

Reference Example 9

3',4',5'-Trimethoxy-2-(3-trimethylsilylpropyloxy)acetophenone (Compound IX-6)

Substantially the same procedure as in Reference Example 5 was repeated using 3',4',5'-trimethoxystyrene oxide (Compound XIIIa, 1.00 g) obtained in Reference Example 2 and 3-trimethylsilylpropanol (18.9 ml) to give 3',4',5'-trimethoxy-2-(3-trimethylsilylpropyloxy)acetophenone (Compound IX-6, 948.4 mg).

$^1$H-NMR (90 MHz, $CDCl_3$) $\delta$ 0.00 (s, 9H), 0.53 (m, 2H), 1.68 (m, 2H), 3.53 (t, J = 6.9 Hz, 2H), 3.92 (s, 9H), 4.67 (s, 2H), 7.27 (s, 2H)

FAB-MS m/z = 341 ($M^{+}$+1)

Reference Example 10

3',4',5'-Trimethoxy-2-(2-methylphenoxy)acetophenone (Compound IX-7)

2-Bromo-3',4',5'-trimethoxyacetophenone (Compound XIa, 1.45g) obtained in Reference Example 1 and 2-methylphenol (648.0 mg) were dissolved in tetrahydrofuran (50 ml), and a 2N aqueous solution of sodium hydroxide (3.0 ml) was added thereto, followed by stirring at room temperature for one hour. The reaction solution was subjected to partitioning between ethyl acetate and water, and the organic layer was successively washed with water and a saturated saline, dried over anhydrous magnesium sulfate, and concentrated under reduced pressure. The residue was purified by silica gel column chromatography to give 3',4',5'-trimethoxy-2-(2-methylphenoxy)acetophenone (Compound IX-7, 0.69 g).

$^{1}$H-NMR (90 MHz, $CDCl_3$) δ 2.30 (s, 3H), 3.90 (s, 6H), 3.93 (s, 3H), 5.20 (s, 2H), 6.76 (dd, J = 6.9, 2.0 Hz, 1H), 6.93 (dd, J = 6.9, 1.6 Hz, 1H), 7.13 (brt, J = 6.9 Hz, 2H), 7.30 (s, 2H)

EI-MS m/z = 316 ($M^{+}$)

Reference Example 11

2-(4-Bromophenoxy)-3',4',5'-trimethoxyacetophenone (Compound IX-8)

Substantially the same procedure as in Reference Example 10 was repeated using 2-bromo-3',4',5'-trimethoxyacetophenone (Compound XIa, 1.45 g) obtained in Reference Example 1 and 4-bromophenol (1.04 g) to give 2-(4-bromophenoxy)-3',4',5'-trimethoxyacetophenone (Compound IX-8, 1.14 g).

$^{1}$H-NMR (90 MHz, $CDCl_3$) δ 3.91 (s, 6H), 3.93 (s, 3H), 5.20 (s, 2H), 6.81 (d, J = 9.0 Hz, 2H), 7.25 (s, 2H), 7.38 (d, J = 9.0 Hz, 2H)

EI-MS m/z = 380, 382 ($M^{+}$)

Reference Example 12

2-Methylthio-3',4',5'-trimethoxyacetophenone (Compound IX-9)

2-Bromo-3',4',5'-trimethoxyacetophenone (Compound XIa, 0.87 g) obtained in Reference Example 1 was dissolved in tetrahydrofuran (60 ml), and a 10% aqueous solution of sodium methanethiolate (3.0 ml) was added thereto, followed by stirring at room temperature for one hour. The reaction solution was subjected to partitioning between ethyl acetate and water, and the organic layer was successively washed with a 5% aqueous solution of sodium bicarbonate and a saturated saline, dried over anhydrous magnesium sulfate, and concentrated under reduced pressure. The residue was purified by silica gel column chromatography to give 3',4',5'-trimethoxy-2-methylthioacetophenone (Compound IX-9, 1.02 g).

$^{1}$H-NMR (90 MHz, $CDCl_3$) δ 2.16 (s, 3H), 3.73 (s, 2H), 3.92 (s, 9H), 7.26 (s, 2H)

EI-MS m/z = 256 ($M^{+}$)

Reference Example 13

3',4',5'-Trimethoxy-2-(2-trimethylsilylethylthio)acetophenone (Compound IX-10)

2-Trimethylsilylethanethiol (1.00 g) was dissolved in tetrahydrofuran (75 ml), and a 1N aqueous solution of sodium hydroxide (7.5 ml) and then 2-bromo-3',4',5'-trimethoxyacetophenone (Compound XIa, 1.79 g) obtained in Reference Example 1 were added thereto, followed by stirring at room temperature for one hour. The reaction solution was subjected to partitioning between ethyl acetate and water, and the organic layer was successively washed with a 5% aqueous solution of sodium bicarbonate and a saturated saline, dried over anhydrous magnesium sulfate, and concentrated under reduced pressure. The residue was purified by silica gel column chromatography to give 3',4',5'-trimethoxy-2-(2-trimethylsilylethylthio)acetophenone (Compound IX-10, 2.07 g).

$^{1}$H-NMR (90 MHz, $CDCl_3$) δ 0.00 (s, 9H), 0.86 (m, 2H), 2.62 (m, 2H), 3.74 (s, 2H), 3.89 (s, 9H), 7.22 (s, 2H)

EI-MS m/z = 342 ($M^{+}$)

Reference Example 14

2-(4-Fluorophenylthio)-3',4',5'-trimethoxyacetophenone (Compound IX-11)

Substantially the same procedure as in Reference Example 13 was repeated using 4-fluorothiophenol (0.82 g) and 2-bromo-3',4',5'-trimethoxyacetophenone (Compound XIa, 1.54 g) obtained in Reference Example 1 to give 2-(4-fluorophenylthio)-3',4',5',-trimethoxyacetophenone (Compound IX-11, 2.24 g).

$^1$H-NMR (90 MHz, $CDCl_3$) δ 3.87 (s, 6H) , 3.92 (s, 3H), 4.15 (s, 2H), 6.97 (t, J = 8.9 Hz, 2H), 7.16 (s, 2H), 7.40 (dd, J = 8.9, 5.3 Hz, 2H)

EI-MS m/z = 336 ($M^+$)

Reference Example 15

2-(2-Hydroxyethylthio)-3',4',5'-trimethoxyacetophenone (Compound IX-12)

Substantially the same procedure as in Reference Example 13 was repeated using 2-mercaptoethanol (0.82 g) and 2-bromo-3',4',5'-trimethoxyacetophenone (Compound XIa, 2.60 g) obtained in Reference Example 1 to give 2-(2-hydroxyethylthio)-3',4',5'-trimethoxyacetophenone (Compound IX-12, 1.98 g).

$^1$H-NMR (90 MHz, $CDCl_3$) δ 2.41 (t, J = 6.0 Hz, 1H), 2.86 (t, J = 6.0 Hz, 2H), 3.82 (q, J = 6.0 Hz, 2H), 3.85 (s, 2H), 3.92 (s, 9H), 7.23 (s, 2H)

EI-MS m/z = 286 ($M^+$)

Reference Example 16

2-(2,3-Dihydroxypropylthio)-3',4',5'-trimethoxyacetophenone (Compound IX-13)

Substantially the same procedure as in Reference Example 13 was repeated using 1-mercapto-2,3-propanediol (12.25 g) and 2-bromo-3',4',5'-trimethoxyacetophenone (Compound XIa, 16.39 g) obtained in Reference Example 1 to give 2-(2,3-dihydroxypropylthio)-3',4',5'-trimethoxyacetophenone (Compound IX-13, 17.00 g).

$^1$H-NMR (270 MHz, $CDCl_3$) δ 2.18 (t, J = 4.0 Hz, 1H), 2.68 (dd, J = 14.1, 7.9 Hz, 1H), 2.79 (dd, J = 14.1, 4.5 Hz, 1H), 3.20 (d, J = 3.5 Hz, 1H), 3.58 (m, 1H), 3.73 (m, 1H), 3.85 (m, 1H), 3.89 (s, 1H), 3.90 (s, 1H), 3.92 (s, 6H), 3.93 (s, 3H), 7.23 (s, 2H)

FAB-MS m/z = 317 ($M^+$+1)

Reference Example 17

2-(β-D-Glucosylthio)-3',4',5'-trimethoxyacetophenone (Compound IX-14)

2-Bromo-3',4',5'-trimethoxyacetophenone (Compound XIa, 289.0 mg) obtained in Reference Example 1 and 1-thio-β-D-glucose sodium salt (290.0 mg) were dissolved in a mixed solvent of tetrahydrofuran (25 ml) and methanol (10 ml), followed by stirring at room temperature for one hour. The reaction mixture was concentrated under reduced pressure and the residue was purified by silica gel column chromatography to give 2-(β-D-glucosylthio)-3',4',5'-trimethoxyacetophenone (Compound IX-14, 390.4 mg).

$^1$H-NMR (270 MHz, DMSO-$d_6$) δ 3.01-3.21 (m, 4H), 3.46 (m, 1H), 3.66 (m, 1H), 3.77 (s, 3H), 3.86 (s, 6H), 4.15 (d, J = 14.8 Hz, 1H), 4.23 (d, J = 14.8 Hz, 1H), 4.33 (d, J = 9.6 Hz, 1H), 4.40 (t, J = 5.9 Hz, 1H), 4.91 (d, J = 4.0 Hz, 1H), 5.03 (d, J = 4.5 Hz, 1H), 5.15 (d, J = 5.5 Hz, 1H), 7.27 (s, 2H)

FAB-MS m/z = 405 ($M^+$+1)

Reference Example 18

2-Carboxymethylthio-3',4',5'-trimethoxyacetophenone (Compound IX-15)

2-Bromo-3',4',5'-trimethoxyacetophenone (Compound XIa, 1.16 g) obtained in Reference Example 1 and thioglycolic acid (460.0 mg) were dissolved in tetrahydrofuran (30 ml), and a 2N aqueous solution of sodium hydroxide (5.0 ml) was added thereto, followed by stirring at room temperature for 16 hours. The reaction solution was subjected to partitioning between chloroform and 1N hydrochloric acid, and the organic layer was dried over anhydrous magnesium sulfate and concentrated under reduced pressure. The residue was purified by silica gel column chromatography to give 2-carboxymethylthio-3',4',5'-trimethoxyacetophenone (Compound IX-15, 0.92 g).

$^1$H-NMR (90 MHz, $CDCl_3$) δ 3.39 (s, 2H), 3.92 (s, 9H), 4.02 (s, 2H), 6.04 (brs, 1H), 7.22 (s, 2H)
FAB-MS m/z = 301 ($M^+$+1)

Reference Example 19

2-(2-Diethylaminoethylthio)-3',4',5'-trimethoxyacetophenone (Compound IX-16)

2-Bromo-3',4',5'-trimethoxyacetophenone (Compound XIa, 1.16 g) obtained in Reference Example 1 and N, N-diethylaminoethanethiol hydrochloride (1.16 g) were dissolved in tetrahydrofuran (30 ml), and a 2N aqueous solution of sodium hydroxide (5.0 ml) was added thereto, followed by stirring at room temperature for 16 hours. The reaction solution was subjected to partitioning between chloroform and water, and the organic layer was dried over anhydrous magnesium sulfate and concentrated under reduced pressure. The residue was purified by silica gel column chromatography to give 2-(2-diethylaminoethylthio)-3',4',5'-trimethoxyacetophenone (Compound IX-16, 1.37 g).

$^1$H-NMR (90 MHz, $CDCl_3$) δ 1.02 (t, J = 7.1 Hz, 6H), 2.54 (q, J = 7.1 Hz, 4H), 2.69 (s, 4H), 3.80 (s, 2H), 3.91 (s, 9H), 7.24 (s, 2H)
EI-MS m/z = 341 ($M^+$)

Reference Example 20

3',4',5',-Trimethoxy-2-(4-phenoxybutylthio)acetophenone (Compound IX-17)

Process 1

4-Phenoxybutyl bromide (5.73 g) was dissolved in dimethyl sulfoxide, and potassium thioacetate (10.71 g) was added thereto, followed by stirring at room temperature for 18 hours. The reaction solution was subjected to partitioning between ethyl acetate and water, and the organic layer was successively washed with water and a saturated saline, dried over anhydrous magnesium sulfate, and concentrated under reduced pressure. The residue was purified by silica gel column chromatography to give 1-acetylthio-4-phenoxybutane (Compound XIX-1, 5.53 g).

$^1$H-NMR (270 MHz, $CDCl_3$) δ 1.70-1.92 (m, 4H), 2.34 (s, 3H), 2.95 (t, J = 7.1 Hz, 2H), 3.97 (t, J = 6.1 Hz, 2H), 6.88 (d, J = 7.4 Hz, 2H), 6.93 (t, J = 8.2 Hz, 1H), 7.28 (dd, J = 8.2, 7.4 Hz, 2H)
FAB-MS m/z = 225 ($M^+$+1)

Process 2

1-Acetylthio-4-phenoxybutane (Compound XIX-1, 1.00 g) obtained in the above Process 1 was dissolved in piperidine (8 ml), followed by stirring at room temperature for 16 hours. Toluene was added to the reaction solution and the mixture was concentrated under reduced pressure. The residue and 2-bromo-3',4',5'-trimethoxyacetophenone (Compound XIa, 1.16 g) obtained in Reference Example 1 were dissolved in a mixed solvent of tetrahydrofuran (10 ml) and methanol (10 ml), and a 1N aqueous solution of sodium hydroxide (9.8 ml) was added thereto, followed by stirring at room temperature for 20 hours: The reaction solution was subjected to partitioning between ethyl acetate and water, and the organic layer was successively washed with water and a saturated saline, dried over anhydrous magnesium sulfate, and concentrated under reduced pressure. The residue was purified by silica gel column chromatography to give 3',4',5'-trimethoxy-2-(4-phenoxybutylthio)acetophenone (Compound IX-17, 353.4 mg) .

$^1$H-NMR (270 MHz, $CDCl_3$) δ 1.73-1.95 (m, 4H), 2.67 (t, J = 6.9 Hz, 2H), 3.77 (s, 2H), 3.92 (s, 6H), 3.93 (s, 3H) , 3.97 (t, J = 5.9 Hz, 2H), 6.88 (dd, J = 8.6, 0.7 Hz, 2H), 6.93 (t, J = 7.6 Hz, 1H), 7.24 (s, 2H), 7.26 (m, 2H)
EI-MS m/z = 390 ($M^+$)

Reference Example 21

5,6-Dihydroxy-1-(3,4,5-trimethoxyphenyl)hexan-1-one (Compound IXh-1)

Process 1

3,4,5-Trimethoxybenzaldehyde (25.48 g) was dissolved in tetrahydrofuran (50 ml), and 4-pentenylmagnesium bromide (a 1M solution in tetrahydrofuran, 200 ml) was added thereto, followed by stirring at room temperature for 30 minutes. 1N Hydrochloric acid was added to the reaction solution and the mixture was extracted with ethyl acetate. The organic layer was washed with a saturated saline, dried over anhydrous magnesium sulfate, and concentrated under reduced pressure to give 1-hydroxy-1-(3,4,5-trimethoxyphenyl)-5-hexene (Compound VIII-1, 34.58 g).

$^1$H-NMR (270 MHz, $CDCl_3$) δ 1.34-1.89 (m, 4H), 2.12 (q, J = 6.8 Hz, 2H), 3.86 (s, 3H), 3.89 (s, 6H), 4.62 (dd, J = 7.3, 5.6 Hz, 1H), 4.96-5.07 (m, 2H), 5.83 (ddt, J = 17.2, 10.2, 6.8 Hz, 1H), 6.59 (s, 2H), OH; not detected

' EI-MS m/z = 266 ($M^+$)

Process 2

1-Hydroxy-1-(3,4,5-trimethoxyphenyl)-5-hexene (Compound VIII-1, 13.30 g) obtained in the above Process 1 was dissolved in acetone (250 ml), and Jones' reagent (18 ml) was added thereto under ice-cooling, followed by stirring for 30 minutes. 2-Propanol (20 ml) was added to the reaction solution, and the solution was concentrated under reduced pressure, followed by partitioning between ethyl acetate and water. The organic layer was successively washed with water and a saturated saline, dried over magnesium sulfate, and concentrated under reduced pressure. The residue was purified by silica gel column chromatography to give 1-(3,4,5-trimethoxyphenyl)-5-hexen-1-one (Compound IXf-1, 10.80 g).

$^1$H-NMR (270 MHz, $CDCl_3$) δ 1.73 (quint, J = 7.0 Hz, 2H), 2.04 (q, J = 7.0 Hz, 2H), 2.83 (t, J = 7.0 Hz, 2H), 3.797 (s, 3H), 3.803 (s, 6H), 4.86-4.98 (m, 2H), 5.72 (ddt, J = 17.1, 10.2, 7.0 Hz, 1H), 7.10 (s, 2H)

EI-MS m/z = 264 ($M^+$)

Process 3

1-(3,4,5-Trimethoxyphenyl)-5- hexen-1-one (Compound IXf-1, 2.64 g) obtained in the above Process 2 was dissolved in chloroform (50 ml), metachloroperbenzoic acid (4.14 g) was added thereto, followed by stirring at room temperature for 32 hours. Insoluble matters were filtered off, and the filtrate was successively washed with a 10% aqueous solution of sodium thiosulfate and a 5% aqueous solution of sodium bicarbonate. The organic layer was dried over anhydrous sodium sulfate and concentrated under reduced pressure, and the residue was purified by silica gel column chromatography to give 5,6-epoxy-1-(3,4,5-trimethoxyphenyl)hexan-1-one (Compound IXg-1, 2.01 g).

$^1$H-NMR (270 MHz, $CDCl_3$) δ 1.57 (m, 1H), 1.77 (m, 1H), 1.93 (quint, J = 7.3 Hz, 2H), 2.50 (dd, J = 5.0, 3.0 Hz, 1H), 2.77 (dd, J = 5.0, 4.0 Hz, 1H), 2.88 (m, 1H), 2.97 (t, J = 7.3 Hz, 2H), 3.92 (s, 9H), 7.23 (s, 2H)

EI-MS m/z = 280 ($M^+$)

Process 4

5,6-Epoxy-1-(3,4,5-trimethoxyphenyl)hexan-1-one (Compound IXg-1, 1.90 g) obtained in the above Process 3 was dissolved in methanol (30 ml), and sodium acetate (5.57 g) was added thereto, followed by heating under reflux for 48 hours. The reaction solution was concentrated under reduced pressure and the residue was purified by silica gel column chromatography to give 5,6-dihydroxy-1-(3,4,5-trimethoxyphenyl)hexan-1-one (Compound IXh-1, 0.87 g).

$^1$H-NMR (90 MHz, $CDCl_3$) δ 1.53 (m, 2H), 1.87 (m, 2H), 2.68 (s, 2H), 3.01 (t, J = 6.7 Hz, 2H), 3.32-3.80 (m, 3H), 3.92 (s, 9H), 7.22 (s, 2H)

EI-MS m/z = 298 ($M^+$)

Reference Example 22

3',4',5'-Trimethoxy-2-[(2S),(3R)-2,3,4-trihydroxybutylthio]acetophenone (Compound IX-18)

Process 1

(-)-2,3-O-Isopropylidene-D-threitol (9.95 g) was dissolved in tetrahydrofuran (270 ml), and sodium hydride (2.70 g, 60% mineral oil dispersion) and then tertbutyldimethylsilyl chloride (10.17 g) were added thereto, followed by stirring at room temperature for one hour. The reaction solution was concentrated under reduced pressure and the residue was purified by silica gel column chromatography to give (2R),(3R)-4-(tert-butyldimethylsilyloxy)-2,3-O-isopropylidene-1,2,3-butanetriol (15.87 g).

$^1$H-NMR (270 MHz, $CDCl_3$) δ 0.09 (s, 6H), 0.90 (s, 9H), 1.40 (s, 3H), 1.42 (s, 3H), 2.93 (dd, J = 8.3, 4.6 Hz, 1H), 3.62-3.83 (m, 3H), 3.83-3.93 (m, 2H), 3.99 (dt, J = 7.5, 4.5 Hz, 1H)

FAB-MS m/z = 277 ($M^+$+1)

Process 2

(2R),(3R)-4-(tert-Butyldimethylsilyloxy)-2,3-O-isopropylidene-1,2,3-butanetriol (15.87 g) obtained in the above Process 1 was dissolved in pyridine (9.3 ml), and toluenesulfonyl chloride (17.5471 g) was added thereto, followed by stirring at room temperature for 18 hours. Tetrahydrofuran (95 ml) and then a 5% aqueous solution of sodium bicarbonate (200 ml) were added to the reaction solution, followed by stirring for 30 minutes. The reaction solution was subjected to partitioning between ethyl acetate and water, and the organic layer was successively washed with a 10% aqueous solution of citric acid and a saturated saline, dried over anhydrous magnesium sulfate, and concentrated under reduced pressure. The residue was purified by silica gel column chromatography to give (2R),(3R)-4-(tert-butyldimethylsilyloxy)-2,3-O-isopropylidene-1-toluenesulfonyloxy-2,3-butanediol (24.96 g).

$^1$H-NMR (270 MHz, $CDCl_3$) δ 0.04 (s, 6H), 0.87 (s, 9H), 1.33 (s, 3H), 1.35 (s, 3H), 2.45 (s, 3H), 3.64 (dd, J = 10.4, 6.1 Hz, 1H), 3.75-3.87 (m, 2H), 4.04-4.25 (m, 3H), 7.34 (d, J = 8.3 Hz, 2H), 7.80 (d, J = 8.3 Hz, 2H)

EI-MS m/z = 430 ($M^+$)

Process 3

(2R),(3R)-4-(tert-Butyldiméthylsilyloxy)-2,3-O-isopropylidene-1-toluenesulfonyloxy-2,3-butanediol (5.62 g) obtained in the above Process 2 was dissolved in dimethyl sulfoxide (64 ml), and potassium thioacetate (5.62 g) was added thereto, followed by stirring at room temperature for 1'8 hours. The reaction solution was subjected to partitioning between ethyl acetate and water, and the organic layer was successively washed with water and a saturated saline, dried over anhydrous magnesium sulfate, and concentrated under reduced pressure. The residue was purified by silica gel column chromatography to give (2S),(3R)-1-acetylthio-4-(tert-butyldimethylsilyloxy)-2,3-O-isopropylidene-2,3-butanediol (4.04 g).

$^1$H-NMR (270 MHz, $CDCl_3$) δ 0.01 (s, 6H), 0.83 (s, 9H), 1.31 (s, 3H), 1.34 (s, 3H), 2.28 (s, 3H) , 3.01 (dd, J = 13.9, 6.6 Hz, 1H), 3.25 (dd, J = 13.9, 4.3 Hz, 1H), 3.61-3.76 (m, 3H), 4.01 (m, 1H)

FAB-MS m/z = 335 ($M^+$+1)

Process 4

(2S),(3R)-1-Acetylthio-4-(tert-butyldimethylsilyloxy)-2,3-O-isopropylidene-2,3-butanediol (3.50 g) obtained in the above Process 3 was dissolved in piperidine (20 ml), followed by stirring at room temperature for 30 minutes under an atmosphere of argon. Toluene (50 ml) was added to the reaction solution and the mixture was concentrated under reduced pressure. The residue was dissolved in toluene (50 ml) and the mixture was concentrated under reduced pressure. The residue was purified by silica gel column chromatography to give (2S),(3R)-4-(tert-butyldimethylsilyloxy)-2,3-dihydroxy-2,3-O-isopropylidene-1-butanethiol (2.81 g).

$^1$H-NMR (270 MHz, $CDCl_3$) δ 0.06 (s, 6H), 0.88 (s, 9H), 1.39 (s, 3H), 1.42 (s, 3H), 1.62 (t, J = 8.2 Hz, 1H), 2.71 (ddd, J = 13.7, 8.0, 5.9 Hz, 1H), 2.82 (ddd, J = 13.7, 8.7, 4.7 Hz, 1H), 3.69 (dd, J = 10.2, 5.9 Hz, 1H), 3.79-3.91 (m, 2H), 4.03 (m, 1H)

FAB-MS m/z = 293 ($M^+$+1)

Process 5

(2S),(3R)-4-(tert-Butyldimethylsilyloxy)-2,3-dihydroxy-2,3-O-isopropylidene-1-butanethiol (2.71 g) obtained in the above Process 4 was dissolved in a mixed solvent of methanol (50 ml) and water (10 ml), and camphorsulfonic acid (1.08 g) was added thereto, followed by heating under reflux for one hour. The reaction solutuion was cooled to room temperature, and a 1N aqueous solution of sodium hydroxide (13.92 ml) and then a solution (50 ml) of 2-bromo-3',4',5'-trimethoxyacetophenone (Compound XIa, 2.68 g) obtained in Reference Example 1 in tetrahydrofuran were added thereto, followed by stirring for one hour. The reaction solution was concentrated under reduced pressure and the residue was purified by silica gel column chromatography to give 3',4',5'-trimethoxy-2-[(2S),(3R)-2,3,4-trihydroxybutylthio]acetophenone (Compound IX-18, 2.60 g).

$^1$H-NMR (270 MHz, $CDCl_3$) δ 2.49 (t, J = 5.6 Hz, 1H), 2.78 (dd, J = 13.9, 8.1 Hz, 1H), 2.85 (dd, J = 13.9, 3.6 Hz, 1H), 2.90 (d, J = 6.6 Hz, 1H), 3.56 (d, J = 4.0 Hz, 1H), 3.66 (m, 1H), 3.74-3.87 (m, 3H), 3.90 (s, 1H), 3.91 (s, 1H), 3.93 (s, 6H), 3.94 (s, 3H), 7.23 (s, 2H)

FAB-MS m/z = 347 ($M^+$+1)

Reference Example 23

3',4',5'-Trimethoxy-2-[(2R),(3S)-2,3,4-trihydroxybutylthio]acetophenone (Compound IX-19)

Process 1

Substantially the same procedure as in Process 1 of Reference Example 22 was repeated using (+)-2,3-O-isopropylidene-L-threitol (5.08 g) and tertbutyldimethylsilyl chloride (5.19 g) to give (2S),(3S)-4-(tert-butyldimethylsilyloxy)-2,3-O-isopropylidene-1,2,3-butanetriol (7.37 g).

$^1$H-NMR (270 MHz, $CDCl_3$) δ 0.09 (s, 6H), 0.90 (s, 9H), 1.40 (s, 3H), 1.42 (s, 3H), 2.41 (dd, J = 7.9, 4.6 Hz, 1H), 3.62-3.83 (m, 3H), 3.83-3.94 (m, 2H), 4.00 (dt, J = 7.7, 4.5 Hz, 1H)

FAB-MS m/z = 277 ($M^+$+1)

Process 2

Substantially the same procedure as in Process 2 of Reference Example 22 was repeated using (2S),(3S)-4-(tert-butyldimethylsilyloxy)-2,3-O-isopropylidene-1,2,3-butanetriol (7.37 g) obtained in the above Process 1 and toluenesulfonyl chloride (8.13 g) to give (2S),(3S)-4-(tert-butyldimethylsilyloxy)-2,3-O-isopropylidene-1-toluenesulfonyloxy-2,3-butanediol (10.57 g).

$^1$H-NMR (270 MHz, $CDCl_3$) δ 0.04 (s, 6H), 0.86 (s, 9H), 1.33 (s, 3H), 1.36 (s, 3H), 2.45 (s, 3H), 3.66 (dd, J = 10.1, 6.1 Hz, 1H), 3.74-3.90 (m, 2H), 4.03-4.29 (m, 3H), 7.34 (d, J = 8.5 Hz, 2H), 7.80 (d, J = 8.5 Hz, 2H)

FAB-MS m/z = 431 ($M^+$+1)

Process 3

Substantially the same procedure as in Process 3 of Reference Example 22 was repeated using (2S),(3S)-4-(tert-butyldimethylsilyloxy)-2,3-O-isopropylidene-1-toluenesulfonyloxy-2,3-butanediol (5.57 g) obtained in the above. Process 2 and potassium thioacetate (5.57 g) to give (2R),(3S)-1-acetylthio-4-(tert-butyldimethylsilyloxy)-2,3-O-isopropylidene-2,3-butanediol (4.18 g).

$^1$H-NMR (270 MHz, $CDCl_3$) δ 0.08 (s, 6H), 0.91 (s, 9H), 1.38 (s, 3H), 1.42 (s, 3H), 2.36 (s, 3H), 3.08 (dd, J = 13.9, 6.3 Hz, 1H), 3.31 (dd, J = 13.9, 4.3 Hz, 1H), 3.68-3.85 (m, 3H), 4.08 (m, 1H)

FAB-MS m/z = 335 ($M^+$+1)

Process 4

Substantially the same procedure as in Process 4 of Reference Example 22 was repeated using (2R),(3S)-1-acetylthio-4-(tert-butyldimethylsilyloxy)-2,3-O-isopropylidene-2,3-butanediol (3.97 g) obtained in the above Process 3 and piperidine (22 ml) to give (2R),(3S)-4-(tert-butyldimethylsilyloxy)-2,3-dihydroxy-2,3-O-isopropylidene-1-butanethiol (3.18 g).

$^1$H-NMR (270 MHz, $CDCl_3$) δ 0.07 (s, 6H), 0. 90 (s, 9H), 1.40 (s, 3H), 1.43 (s, 3H), 1.63 (t, J = 8.4 Hz, 1H), 2.72 (ddd, J = 13.9, 8.2, 5.9 Hz, 1H), 2.84(ddd, J = 13.9, 8.4, 4.1 Hz, 1H), 3.70 (dd, J = 10.2, 5.9 Hz, 1H), 3.79-3.94 (m, 2H), 4.04 (m, 1H)

FAB-MS m/z = 293 ($M^+$+1)

Process 5

Substantially the same procedure as in Process 5 of Reference Example 22 was repeated using (2R),(3S)-4-(tert-butyldimethylsilyloxy)-2,3-dihydroxy-2,3-O-isopropylidene-1-butanethiol (2.97 g) obtained in the above Process 3 and 2-bromo-3',4',5'-trimethoxyacetophenone (Compound XIa, 2.94 g) obtained in Reference Example 1 to give 3',4',5'-trimethoxy2-[(2R),(3S)-2,3,4-trihydroxybutylthio]acetophenone (Compound IX-19, 3.11 g).

$^1$H-NMR (270 MHz, $CDCl_3$) δ 2.53 (t, J = 5.9 Hz, 1H), 2.77 (dd, J = 13.5, 7.4 Hz, 1H), 2.85 (dd, J = 13.5, 3.6 Hz, 1H), 2.94 (d, J = 6.6 Hz, 1H), 3.58 (d, J = 4.0 Hz, 1H), 3.65 (m, 1H), 3.74-3.87 (m, 3H), 3.90 (s, 1H), 3.91 (s, 1H), 3.92 (s, 6H), 3.94 (s, 3H), 7.23 (s, 2H)

FAB-MS m/z = 347 ($M^+$+1)

Reference Example 24

Indole-5-carbaldehyde

Indole-5-carboxylic acid (3.10 g) was dissolved in tetrahydrofuran (50 ml), and lithium aluminium hydride (1.50 g) was added thereto, followed by heating under reflux for 20 hours. The reaction solution was cooled to room temperature, and ethyl acetate and then a 2N aqueous solution of sodium hydroxide were added thereto to cease the reaction. The reaction solution was dried over anhydrous sodium sulfate and insoluble matters were filtered off. Manganese dioxide (30.00 g) was added to the filtrate, followed by stirring at room temperature for 96 hours. Insoluble matters were filtered off and the filtrate was concentrated under reduced pressure. The residue was purified by silica

gel column chromatography to give indole-5-carbaldehyde (1.43 g).

$^1$H-NMR (90 MHz, $CDCl_3$) δ 6.72 (m, 1H), 7.33 (t, J = 3.0 Hz, 1H), 7.49 (d, J = 8.6 Hz, 1H), 7.97 (dd, J = 8.6, 1.3 Hz, 1H), 8.19 (brs, 1H), 8.57 (brs, 1H), 10.04 (s, 1H)

EI-MS m/z = 145 ($M^+$)

Example 1

(Z)-3-(Indol-3-yl)-2-methoxy-1-(3,4,5-trimethoxyphenyl)-2-propen-1-one (Compound 1)

2-Methoxy-3',4',5'-trimethoxyacetophenone (300.0 mg) obtained in Reference Example 4 and indole-3-carbaldehyde (362.5 mg) were dissolved in ethanol (10 ml), and piperidine (212.9 mg) was added thereto, followed by heating under reflux for 9 hours. The reaction solution was ice-cooled and the precipitated crystals were collected by filtration. The obtained crude crystals were recrystallized from ethyl acetate to give Compound 1 (254.8 mg).

$^1$H-NMR (270 MHz, $CDCl_3$) δ 3.88 (s, 3H), 3.92 (s, 6H), 3.97 (s, 3H), 7.11 (s, 1H), 7.15-7.29 (m, 2H), 7.19 (s, 2H), 7.43 (dd, J = 7.6, 1.3 Hz, 1H), 7.65 (d, J = 7.6 Hz, 1H), 8.11 (d, J = 2.6 Hz, 1H), 8.67 (brs, 1H)

EI-MS m/z = 367 ($M^+$)

| Elemental Analysis: $C_{21}H_{21}NO_5$ | | | |
|---|---|---|---|
| Calcd.(%) | C, 68.65; | H, 5.76; | N, 3.81 |
| Found (%) | C, 68.60; | H, 5.68; | N, 3.75 |

Example 2

(Z)-2-Ethoxy-3-(indol-3-yl)-1-(3,4,5-trimethoxyphenyl)-2-propen-1-one (Compound 2)

2-Ethoxy-3',4',5'-trimethoxyacetophenone (509.0 mg) obtained in Reference Example 5 and indole-3-carbaldehyde (435.0 mg) were dissolved in ethanol (12 ml), and piperidine (255.5 mg) was added thereto, followed by heating under reflux for 24 hours. The reaction solution was concentrated under reduced pressure and the residue was purified by silica gel column chromatography. The obtained crude crystals were recrystallized from a mixed solvent of ethyl acetate and hexane (1:1) to give Compound 2 (112.5 mg).

$^1$H-NMR (270 MHz, $CDCl_3$) δ 1.42 (t, J = 7.0 Hz, 3H), 3.92 (s, 6H), 3.96 (s, 3H), 4.11 (q, J = 7.0 Hz, 2H), 7.10 (s, 1H), 7.15-7.32 (m, 2H), 7.21 (s, 2H), 7.43 (d, J = 8.6 Hz, 1H), 7.66 (d, J = 7.6 Hz, 1H), 8.17 (d, J = 2.6 Hz, 1H), 8.58 (brs, 1H).

EI-MS m/z = 381 ($M^+$)

| Elemental Analysis: $C_{22}H_{23}NO_5$ | | | |
|---|---|---|---|
| Calcd.(%) | C, 69.28; | H, 6.08; | N, 3.67 |
| Found (%) | C, 69.52; | H, 6.27; | N, 3.57 |

Example 3

(Z)-3-(Indol-3-yl)-2-propyloxy-1-(3,4,5-trimethoxyphenyl)-2-propen-1-one (Compound 3)

3',4',5'-Trimethoxy-2-propyloxyacetophenone (690.0 mg) obtained in Reference Example 6 and indole-3-carbaldehyde (373.3 mg) were dissolved in ethanol (10 ml), and piperidine (254.6 mg) was added thereto, followed by heating under reflux for 24 hours. The reaction solution was concentrated under reduced pressure and the residue was purified by silica gel column chromatography. The obtained crude crystals were recrystallized from a mixed solvent of ethyl acetate, hexane, and isopropyl ether (2:2:3) to give Compound 3 (701.5 mg).

$^1$H-NMR (270 MHz, $CDCl_3$) δ 1.02 (t, J = 7.2 Hz, 3H), 1.82 (sext, J = 7.2 Hz, 2H), 3.92 (s, 6H), 3.96 (s, 3H), 4.01 (t, J = 7.2 Hz, 2H), 7.05 (s, 1H), 7.16-7.31 (m, 2H), 7.21 (s, 2H), 7.43 (d, J = 7.9 Hz, 1H), 7.64 (d, J = 7.9 Hz, 1H), 8.15 (d, J = 2.5 Hz, 1H), 8.57 (brs, 1H)

EI-MS m/z = 395 ($M^+$)

| Elemental Analysis: $C_{23}H_{25}NO_5$ | | | |
|---|---|---|---|
| Calcd.(%) | C, 69.86; | H, 6.37; | N, 3.54 |
| Found (%) | C, 69.88; | H, 6.45; | N, 3.53 |

Example 4

(Z)-2-Isopropyloxy-3-(indol-3-yl)-1-(3,4,5-trimethoxyphenyl)-2-propen-1-one (Compound 4)

2-Isopropyloxy-3',4',5'-trimethoxyacetophenone (443.0 mg) obtained in Reference Example 7 and indole-3-carbaldehyde (360.0 mg) were dissolved in ethanol (10 ml), and piperidine (211.0 mg) was added thereto, followed by heating under reflux for 48 hours. The reaction solution was concentrated under reduced pressure and the residue was purified by silica gel column chromatography. The obtained crude crystals were recrystallized from a mixed solvent of ethyl acetate and hexane (1:1) to give Compound 4 (386.0 mg).

$^1$H-NMR (270 MHz, $CDCl_3$) δ 1.34 (d, J = 6.2 Hz, 6H), 3.92 (s, 6H), 3.96 (s, 3H), 4.51 (m, 1H), 7.09 (s, 1H), 7.15-7.29 (m, 2H), 7.23 (s, 2H), 7.42 (dd, J = 7.3, 1.0 Hz, 1H), 7.66 (d, J = 7.6 Hz, 1H), 8.21 (d, J = 2.6 Hz, 1H), 8.54 (brs, 1H)

EI-MS m/z = 395 ($M^+$)

| Elemental Analysis: $C_{23}H_{25}NO_5$ | | | |
|---|---|---|---|
| Calcd.(%) | C, 69.86; | H, 6.37; | N, 3.54 |
| Found (%) | C, 69.88; | H, 6.57; | N, 3.50 |

Example 5

(Z)-3-(Indol-3-yl)-1-(3,4,5-trimethoxyphenyl)-2-(2-trimethylsilylethoxy)-2-propen-1-one (Compound 5)

3',4',5'-Trimethoxy-2-(2-trimethylsilylethoxy)acetophenone (720.0 mg) obtained in Reference Example 8 and indole-3-carbaldehyde (640.0 mg) were dissolved in ethanol (15 ml), and piperidine (376.1 mg) was added thereto, followed by heating under reflux for 24 hours. The reaction solution was concentrated under reduced pressure and the residue was purified by silica gel column chromatography. The obtained crude crystals were purified by preparative high pressure liquid chromatography (HPLC) (YMC pack ODS, SH-343-5, S-5, 120A, 250 x 20 mm, acetonitrile:water = 80:20) to give Compound 5 (83.7 mg).

$^1$H-NMR (270 MHz, $CDCl_3$) δ 0.01 (s, 9H), 1.23 (m, 2H), 3.92 (s, 6H), 3.96 (s, 3H), 4.14 (m, 2H), 7.07 (s, 1H), 7.20 (s, 2H), 7.22 (m, 1H), 7.28 (m, 1H), 7.43 (dd, J = 8.1, 0.9 Hz, 1H), 7.64 (d, J = 7.3 Hz, 1H), 8.22 (d, J = 2.6 Hz, 1H), 8.57 (brs, 1H)

EI-MS m/z = 453 ($M^+$)

| Elemental Analysis: $C_{25}H_{31}NO_5Si$ | | | |
|---|---|---|---|
| Calcd.(%) | C, 66.20; | H, 6.89; | N, 3.09 |
| Found (%) | C, 66.14; | H, 6.72; | N, 3.15 |

Example 6

(Z)-3-(Indol-3-yl)-1-(3,4,5-trimethoxyphenyl)-2-(2-trimethylsilylpropyloxy)-2-propen-1-one (Compound 6)

3',4',5'-Trimethoxy-2-(3-trimethylsilylpropyloxy)acetophenone(843.0 mg) obtained in Reference Example 9 and indole-3-carbaldehyde (359.5 mg) were dissolved in ethanol (10 ml), and piperidine (211.1 mg) was added thereto, followed by heating under reflux for 24 hours. The reaction solution was concentrated under reduced pressure and the residue was purified by silica gel column chromatography. The obtained crude crystals were recrystallized from a mixed solvent of ethyl acetate, hexane, and isopropyl ether (2:2:3) to give Compound 6 (547.1 mg).

$^1$H-NMR (270 MHz, $CDCl_3$) δ -0.01 (s, 9H), 0.57 (m, 2H), 1.80 (m, 2H), 3.93 (s, 6H), 3.97 (s, 3H), 4.01 (m, 2H), 7.05 (s, 1H), 7.16-7.33 (m, 2H), 7.21 (s, 2H), 7.44 (d, J = 7.4 Hz, 1H), 7.66 (d, J = 7.9 Hz, 1H), 8.16 (d, J = 3.0 Hz, 1H), 8.56 (s, 1H)

EI-MS m/z = 467 ($M^+$)

| Elemental Analysis: $C_{26}H_{33}NO_5Si$ | | | |
|---|---|---|---|
| Calcd.(%) | C, 66.78; | H, 7.11; | N, 3.00 |
| Found (%) | C, 66.77; | H, 7.30; | N, 2.94 |

Example 7

(Z)-3-(Indol-3-yl)-2-(2-methylphenoxy)-1-(3,4,5-trimethoxyphenyl)-2-propen-1-one (Compound 7)

3',4',5'-Trimethoxy-2-(2-methylphenoxy)acetophenone (600.0 mg) obtained in Reference Example 10 and indole-3-carbaldehyde (551.0 mg) were dissolved in ethanol (20 ml), and piperidine (323.0 mg) was added thereto, followed by heating under reflux for 48 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:water = 70:30) to give Compound 7 (601.4 mg).

$^1$H-NMR (270 MHz,. $CDCl_3$) δ 2.49 (s, 3H), 3.88 (s, 6H), 3.92 (s, 3H), 6.81 (dd, J = 7.9, 1.3 Hz, 1H), 6.89 (dd, J = 7.3, 1.3 Hz, 1H), 7.00 (ddd, J = 7.9, 7.3, 1.3 Hz, 1H), 7.15 (s, 2H), 7.19 (dd, J = 7.3, 1.3 Hz, 1H), 7.23-7.30 (m, 2H), 7.40 (m, 1H), 7.61 (s, 1H), 7.73 (m, 1H), 7.86 (d, J = 2.6 Hz, 1H), 8.54 (brs, 1H)

EI-MS m/z = 443 ($M^+$)

| Elemental Analysis: $C_{27}H_{25}NO_5$ | | | |
|---|---|---|---|
| Calcd.(%) | C, 73.12; | H, 5.68; | N, 3.16 |
| Found (%) | C, 72.87; | H, 5.80; | N, 2.99 |

Example 8

(Z)-2-(4-Bromophenoxy)-3-(indol-3-yl)-1-(3,4,5-trimethoxyphenyl)-2-propen-1-one (Compound 8)

2-(4-Bromophenoxy)-3',4',5'-trimethoxyacetophenone (906.8 mg) obtained in Reference Example 11 and indole-3-carbaldehyde (652.5 mg) were dissolved in ethanol (15 ml), and piperidine (382.5 mg) was added thereto, followed by heating under reflux for 24 hours. The reaction solution was concentrated under reduced pressure and the residue was purified by silica gel column chromatography to give Compound 8 (1.18 g).

$^1$H-NMR (270 MHz, $CDCl_3$) δ 3.90 (s, 6H), 3.94 (s, 3H), 6.92 (d, J = 9.1 Hz, 2H), 7.18 (s, 2H), 7.21-7.32 (m, 2H), 7.35 (d, J = 9.1 Hz, 2H), 7.42 (dd, J = 6.6, 2.0 Hz, 1H), 7.69 (s, 1H), 7.73 (dd, J = 6.2, 2.1 Hz, 1H), 7.83 (d, J = 3.0 Hz, 1H), 8.59 (brs, 1H)

EI-MS m/z = 507, 509 ($M^+$)

| Elemental Analysis: $C_{26}H_{22}BrNO_5$ | | | |
|---|---|---|---|
| Calcd.(%) | C, 61.43; | H, 4.36; | N, 2.76 |
| Found (%) | C, 61.33; | H, 4.41; | N, 2.53 |

Example 9

(Z)-3-(Indol-3-yl)-2-methylthio-1-(3,4,5-trimethoxyphenyl)-2-propen-1-one (Compound 9)

2-Methylthio-3',4',5'-trimethoxyacetophenone (900.0 mg) obtained in Reference Example 12 and indole-3-carbaldehyde (1.02 g) were dissolved in ethanol (35 ml), and piperidine (598.4 mg) was added thereto, followed by heating under reflux for 48 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:water = 70:30) to give Compound 9 (752.9 mg).

$^1$H-NMR (270 MHz, $CDCl_3$) δ 2.39 (s, 3H), 3.90 (s, 6H), 3.97 (s, 3H), 7.16-7.30 (m, 2H), 7.20 (s, 2H), 7.44 (d, J = 7.9 Hz, 1H), 7.58 (d, J = 7.9 Hz, 1H), 7.67 (s, 1H), 8.33 (d, J = 2.6 Hz, 1H), 8.73 (brs, 1H)

EI-MS m/z = 383 ($M^+$)

| Elemental Analysis: $C_{21}H_{21}NO_4S$ | | | |
|---|---|---|---|
| Calcd.(%): | C, 65.78; | H, 5.52; | N, 3.65 |
| Found (%): | C, 66.04; | H, 5.37; | N, 3.58 |

Example 10

(Z)-3-(Indol-3-yl)-1-(3,4,5-trimethoxyphenyl)-2-(2-trimethylsilylethylthio)-2-propen-1-one (Compound 10)

3',4',5'-Trimethoxy-2-(2-trimethylsilylethylthio)acetophenone (1.00 g) obtained in Reference Example 13 and indole-3-carbaldehyde (0.85 g) were dissolved in ethanol (30 ml), and piperidine (0.50 g) was added thereto, followed by heating under reflux for 48 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:water = 80:20) to give Compound 10 (0.76 g).

$^1$H-NMR (270 MHz, $CDCl_3$) δ -0.05 (s, 9H), 0.89 (m, 2H), 2.90 (m, 2H), 3.89 (s, 6H), 3.97 (s, 3H), 7.16-7.30 (m, 2H), 7.20 (s, 2H), 7.44 (d, J = 7.9 Hz, 1H), 7.60 (d, J = 7.6 Hz, 1H), 7.71 (s, 1H), 8.39 (d, J = 3.0 Hz, 1H), 8.67 (brs, 1H)

EI-MS m/z = 469 ($M^+$)

| Elemental Analysis: $C_{25}H_{31}NO_4SSi$ | | | |
|---|---|---|---|
| Calcd.(%) | C, 63.93; | H, 6.65; | N, 2.98 |
| Found (%) | C, 63.95; | H, 6.70; | N, 2.92 |

Example 11

(Z)-2-(4-Fluorophenylthio)-3-(indol-3-yl)-1-(3,4,5-trimethoxyphenyl)-2-propen-1-one (Compound 11)

2-(4-Fluorophenylthio)-3',4',5'-trimethoxyacetophenone (1.19 g) obtained in Reference Example 14 and indole-3-carbaldehyde (1.03 g) were dissolved in ethanol (35 ml), and piperidine (0.60 g) was added thereto, followed by heating under reflux for 24 hours. The reaction solution was concentrated under reduced pressure and the residue was purified by silica gel column chromatography. The obtained crude crystals were recrystallized from a mixed solvent of ethyl acetate and hexane (1:1) to give Compound 11 (0.83 g).

$^1$H-NMR (270 MHz, $CDCl_3$) δ 3.84 (s, 6H), 3.93 (s, 3H), 6.89 (t, J = 8.6 Hz, 2H), 7.03 (s, 2H), 7.21-7.33 (m, 4H), 7.44 (d, J = 7.6 Hz, 1H), 7.69 (d, J = 7.3 Hz, 1H), 8.01 (s, 1H), 8.40 (d, J = 3.0 Hz, 1H), 8.78 (brs, 1H)

EI-MS m/z = 463 ($M^+$)

| Elemental Analysis: $C_{26}H_{22}FNO_4S$ | | | |
|---|---|---|---|
| Calcd.(%) | C, 67.37; | H, 4.78; | N, 3.02 |
| Found (%) | C, 67.32; | H, 4.82; | N, 2.95 |

Example 12

(Z)-2-(2-Hydroxyethylthio)-3-(indol-3-yl)-1-(3,4,5-trimethoxyphenyl)-2-propen-1-one (Compound 12)

2-(2-Hydroxyethylthio)-3',4',5'-trimethoxyacetophenone (858.0 mg) obtained in Reference Example 15 and indole-3-carbaldehyde (435.0 mg) were dissolved in ethanol (10 ml), and piperidine (255.0 mg) was added thereto, followed by heating under reflux for 24 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:water = 50:50) to give Compound 12 (636.5 mg).

$^1$H-NMR (270 MHz, $CDCl_3$) δ 3.04 (t, J = 5.6 Hz, 2H), 3.16 (t, J = 5.6 Hz, 1H), 3.71 (q, J = 5.6 Hz, 2H), 3.88 (s, 6H), 3.97 (s, 3H), 7.12 (s, 2H), 7.20-7.32 (m, 2H), 7.46 (d, J = 7.9 Hz, 1H), 7.56 (d, J = 7.9 Hz, 1H), 7.95 (s, 1H), 8.60 (d, J = 3.0 Hz, 1H), 8.88 (brs, 1H)

FAB-MS m/z = 414 ($M^+$+1)

| Elemental Analysis: $C_{22}H_{23}NO_5S \cdot 0.5H_2O$ | | | |
|---|---|---|---|
| Calcd.(%) | C, 62.54; | H, 5.73; | N, 3.32 |
| Found (%) | C, 62.56; | H, 5.55; | N, 3.02 |

Example 13

(Z)-2-(2-Hydroxyethylthio)-3-(6-methylindol-3-yl)-1-(3,4,5-trimethoxyphenyl)-2-propen-1-one (Compound 13)

2-(2-Hydroxyethylthio)-3',4',5'-trimethoxyacetophenone (1.00 g) obtained in Reference Example 15 and 6-methylindole-3-carbaldehyde [J. Org. Chem., 44, 3741 (1979)] (0.56 g) were dissolved in ethanol (10 ml), and piperidine (0.30 g) was added thereto, followed by heating under reflux for 72 hours. The reaction solution was concentrated under reduced pressure and the residue was purified by silica gel column chromatography. The obtained crude crystals were recrystallized from a mixed solvent of ethyl acetate and hexane (1:1) to give Compound 13 (911.3 mg).

$^1$H-NMR (270 MHz, $CDCl_3$) δ 2.47 (s, 3H), 3.03 (t, J = 5.4 Hz, 2H), 3.09 (s, 1H), 3.70 (t, J = 5.4 Hz, 2H), 3.88 (s, 6H), 3.97 (s, 3H), 7.04 (d, J = 8.4 Hz, 1H), 7.12 (s, 2H), 7.24 (s, 1H), 7.42 (d, J = 8.4 Hz, 1H), 7.94 (s, 1H), 8.53 (d, J = 2.5 Hz, 1H), 8.75 (brs, 1H)

EI-MS m/z = 427 ($M^+$)

| Elemental Analysis: $C_{23}H_{25}NO_5S$ | | | |
|---|---|---|---|
| Calcd.(%) | C, 64.62; | H, 5.89; | N, 3.28 |
| Found (%) | C, 64.37; | H, 6.13; | N, 3.14 |

Example 14

(Z)-2-(β-D-Glucosylthio)-3-(6-methylindol-3-yl)-1-(3,4,5-trimethoxyphenyl)-2-propen-1-one (Compound 14)

2-(β-D-Glucosylthio)-3',4',5'-trimethoxyacetophenone (1.01 g) obtained in Reference Example 17 and 6-methylindole-3-carbaldehyde (0.40 g) were dissolved in ethanol (10 ml), and piperidine (0.21 g) was added thereto, followed by heating under reflux for 24 hours. The reaction solution was concentrated under reduced pressure and the residue was purified by silica gel column chromatography. The obtained crude crystals were recrystallized from ethanol and purified by preparative HPLC (YMC pack ODS, SH-343-5, S-5, 120A, 250 x 20 mm, acetonitrile:water = 40:60). The eluate was concentrated under reduced pressure and the residue was recrystallized from a mixed solvent of ethanol and isopropyl ether (1:1) to give Compound 14 (322.2 mg).

$^1$H-NMR (270 MHz, DMSO-$d_6$) δ 2.40 (s, 3H), 2.78 (m, 1H), 3.16-3.32 (m, 5H), 3.79 (s, 3H), 3.80 (s, 3H), 3.81 (s, 3H), 4.24 (t, J = 5.6 Hz, 1H), 4.76 (d, J = 8.6 Hz, 1H), 4.82 (d, J = 4.6 Hz, 1H), 5.09 (d, J = 2.9 Hz, 1H), 5.46 (d, J = 5.0 Hz, 1H), 6.94 (d, J = 8.3 Hz, 1H), 7.09 (s, 2H), 7.26 (s, 1H), 7.33 (d, J = 8.3 Hz, 1H), 7.52 (s, 1H), 8.12 (d, J = 2.5 Hz, 1H), 11.73 (s, 1H)

FAB-MS m/z = 545 ($M^+$+1)

| Elemental Analysis: $C_{27}H_{31}NO_9S \cdot 0.8H_2O$ | | | |
|---|---|---|---|
| Calcd.(%) | C, 57.91; | H, 5.87; | N, 2.50 |
| Found (%) | C, 57.88; | H, 5.77; | N, 2.40 |

Example 15

(Z)-2-Carboxymethylthio-3-(indol-3-yl)-1-(3,4,5-trimethoxyphenyl)-2-propen-1-one (Compound 15)

2-Carboxymethylthio-3',4',5'-trimethoxyacetophenone (0.60 g) obtained in Reference Example 18 and indole-3-carbaldehyde (0.29 g) were dissolved in ethanol (5 ml), and piperidine (0.34 g) was added thereto, followed by heating under reflux for 40 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:a 0.1M aqueous solution of ammonium acetate = 30:70). The eluate was concentrated under reduced pressure and the residue was subjected to partitioning between chloroform and a 10% aqueous solution of citric acid. The organic layer was concentrated under reduced pressure to give Compound 15 (343.3 mg).

$^1$H-NMR (270 MHz, $CDCl_3$) δ 3.62 (s, 2H) , 3.88 (s, 6H), 3.99 (s, 3H), 7.11 (s, 2H), 7.21 (m, 1H), 7.31 (m, 1H), 7.47 (d, J = 7.9 Hz, 1H), 7.52 (d, J = 7.9 Hz, 1H), 7.99 (s, 1H), 8.52 (d, J = 3.0 Hz, 1H), 9.10 (brs, 1H), CO2H; not detected

FAB-MS m/z = 428 ($M^+$+1)

| Elemental Analysis: $C_{22}H_{21}NO_6S \cdot 0.4H_2O$ | | | |
|---|---|---|---|
| Calcd.(%) | C, 60.79; | H, 5.05; | N, 3.22 |
| Found (%) | C, 60.76; | H, 4.86; | N, 3.17 |

Example 16

(Z)-3-(Indol-3-yl)-2-methoxycarbonylmethylthio-1-(3,4,5-trimethoxyphenyl)-2-propen-1-one (Compound 16)

Compound 15 (50.0 mg) obtained in Example 15 was dissolved in a mixed solvent of chloroform (10 ml) and methanol (5 ml), and a solution (0.5 ml) of trimethylsilyldiazomethane in hexane, followed by stirring for 10 minutes. Acetic acid (20.0 mg) was added to the reaction solution and the mixture was subjected to partitioning between chloroform and a 5% aqueous solution of sodium bicarbonate. The organic layer was concentrated under reduced pressure and the residue was purified by silica gel column chromatography. The obtained crude crystals were recrystallized from isopropyl ether to give Compound 16 (36.9 mg).

$^1$H-NMR (270 MHz, $CDCl_3$) δ 3.58 (s, 3H), 3.68 (s, 2H), 3.90 (s, 6H), 3.97 (s, 3H), 7.15 (s, 2H), 7.19 (m, 1H), 7.28 (m, 1H), 7.48 (m, 1H), 7.54 (d, J = 7.9 Hz, 1H), 7.83 (s, 1H), 8.46 (d, J = 2.7 Hz, 1H), 8.78 (s, 1H)

EI-MS m/z = 441 ($M^+$)

| Elemental Analysis: $C_{23}H_{23}NO_6S$ | | | |
|---|---|---|---|
| Calcd.(%) | C, 62.57; | H, 5.25; | N, 3.17 |
| Found (%) | C, 62.40; | H, 5.26; | N, 3.13 |

Example 17

(Z)-2-(2-Diethylaminoethylthio)-3-(indol-3-yl)-1-(3,4,5-trimethoxyphenyl)-2-propen-1-one (Compound 17)

2-(2-Diethylaminoethylthio)-3',4',5'-trimethoxyacetophenone (671.0 mg) obtained in Reference Example 19 and indole-3-carbaldehyde (290.0 mg) were dissolved in ethanol (5 ml), and piperidine (170.0 mg) was added thereto, followed by heating under reflux for 44 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:a 0.1M aqueous solution of ammonium-acetate = 50:50). The eluate was concentrated under reduced pressure and the residue was subjected to partitioning between chloroform and a 5% aqueous solution of sodium bicarbonate. The organic layer was concentrated under reduced pressure to give Compound 17 (410.6 mg).

$^1$H-NMR (270 MHz, $CDCl_3$) δ 0.95 (t, J = 7.1 Hz, 6H), 2.49 (q, J = 7.1 Hz, 4H), 2.71 (m, 2H), 2.94 (m, 2H), 3.89 (s, 6H), 3.97 (s, 3H), 7.18 (s, 2H), 7.19 (m, 1H), 7.27 (m, 1H), 7.44 (d, J = 7.9 Hz, 1H), 7.57 (d, J = 7.6 Hz, 1H), 7.73 (s, 1H), 8.43 (d, J = 2.3 Hz, 1H), 8.87 (s, 1H)

FAB-MS m/z = 469 ($M^+$+1)

| Elemental Analysis: $C_{26}H_{32}N_2O_4S \cdot 0.6H_2O$ | | | |
|---|---|---|---|
| Calcd.(%) | C, 65.14; | H, 6.98; | N, 5.84 |
| Found (%) | C, 65.25; | H, 7.16; | N, 5.86 |

Example 18

(Z)-3-(Indol-3-yl)-2-(4-phenoxybutylthio)-1-(3,4,5-trimethoxyphenyl)-2-propen-1-one (Compound 18)

2-(4-Phenoxybutylthio)-3',4',5'-trimethoxyacetophenone (195.0 mg) obtained in Reference Example 20 and indole-3-carbaldehyde(72.5 mg) were dissolved in ethanol (3 ml), and piperidine (42.6 mg) was added thereto, followed by heating under reflux for 4 hours. The reaction solution was concentrated under reduced pressure and the residue was purified by silica gel column chromatography. The obtained crude crystals were purified by preparative HPLC (YMC pack ODS, SH-343-5, S-5, 120A, 250 x 20 mm, acetonitrile:water = 70:30). The eluate was concentrated under reduced pressure and the the obtained crude crystals were recrystallized from isopropyl ether to give Compound 18 (94.0 mg).

$^1H$-NMR (270 MHz, $CDCl_3$) δ 1.71-1.93 (m, 4H), 2.95 (t, J = 7.1 Hz, 2H), 3.87 (s, 6H), 3.89 (t, J = 6.9 Hz, 2H), 3.97 (s, 3H), 6.77 (d, J = 7.9 Hz, 2H), 6.89 (t, J = 7.3 Hz, 1H), 7.16-7.31 (m, 4H), 7.18 (s, 2H), 7.44 (d, J = 8.3 Hz, 1H), 7.58 (d, J = 7.9 Hz, 1H), 7.75 (s, 1H), 8.42(d, J = 1.0 Hz, 1H), 8.68 (brs, 1H)

EI-MS m/z = 517 ($M^+$)

| Elemental Analysis: $C_{30}H_{31}NO_5S$ | | | |
|---|---|---|---|
| Calcd.(%) | C, 69.61; | H, 6.04; | N, 2.71 |
| Found (%) | C, 69.57; | H, 6.36; | N, 2.72 |

Example 19

(E)-2-(3,4-Dihydroxybutyl)-3-(indol-3-yl)-1-(3,4,5-trimethoxyphenyl)-2-propen-1-one (Compound 19)

5,6-Dihydroxy-1-(3,4,5-trimethoxyphenyl)hexan-1-one(787.2 mg) obtained in Reference Example 21 and indole-3-carbaldehyde(382.8 mg) were dissolved in ethanol (7 ml), and piperidine (261.1 mg) was added thereto, followed by heating under reflux for 48 hours. N,N,N'-Trimethylethylenediamine (269.8 mg) was added to the reaction solution and the mixture was heated under reflux further for 48 hours. The reaction solution was concentrated under reduced pressure and the residue was purified by silica gel column chromatography. The obtained crude crystals were recrystallized from a mixed solvent of ethyl acetate and hexane (3:2) and then from ethanol to give Compound 19 (98.4 mg).

$^1H$-NMR (270 MHz, $CDCl_3$) δ 1.67-1.94 (m, 2H), 2.33 (brt, J = 5.8 Hz, 1H), 2.89 (m, 1H), 3.03 (m, 1H), 3.54 (m, 1H), 3.67 (m, 1H), 3.78 (m, 1H), 3.88 (s, 6H), 3.96 (brs, 1H), 3.97 (s, 3H), 7.00 (s, 2H), 7.17-7.31 (m, 2H), 7.44 (d, J = 7.9 Hz, 1H), 7.54 (d, J = 7.9 Hz, 1H), 7.77 (s, 1H), 7.87 (d, J = 2.6 Hz, 1H), 8.92 (brs, 1H)

FAB-MS m/z = 426 ($M^+$+1)

| Elemental Analysis: $C_{24}H_{27}NO_6 \cdot 0.5H_2O$ | | | |
|---|---|---|---|
| Calcd.(%) | C, 66.35; | H, 6.50; | N, 3.22 |
| Found (%) | C, 66.40; | H, 6.67; | N, 3.18 |

Example 20

(E)-2-(3,4-Dihydroxybutyl)-3-(6-methylindol-3-yl)-1-(3,4,5-trimethoxyphenyl)-2-propen-1-one (Compound 20)

5,6-Dihydroxy-1-(3,4,5-trimethoxyphenyl)hexan-1-one (870.0 mg) obtained in Reference Example 21 and 6-methylindole-3-carbaldehyde (465.0 mg) were dissolved in ethanol (8 ml), and piperidine (288.8 mg) was added thereto, followed by heating under reflux for 48 hours. The reaction solution was concentrated under reduced pressure and the residue was purified by silica gel column chromatography. The obtained crude crystals were recrystallized from a mixed solvent of ethyl acetate and hexane (5:3) and then from a mixed solvent of ethanol and water (1:1) to give Compound 20 (142.7 mg).

$^1H$-NMR (270 MHz, $CDCl_3$) δ 1.70-1.95 (m, 2H), 2.36 (brs, 1H), 2.47 (s, 3H), 2.87 (m, 1H), 3.03 (m, 1H), 3.54 (m, 1H), 3.65 (m, 1H), 3.77 (m, 1H), 3.88 (s, 6H), 3.96 (s, 3H), 4.04 (brs, 1H), 7.00 (s, 2H), 7.02 (d, J = 8.8 Hz, 1H), 7.22 (s, 1H), 7.41 (d, J = 8.8 Hz, 1H), 7.75 (s, 1H), 7.79 (d, J = 2.3 Hz, 1H), 8.79 (s, 1H)

FAB-MS m/z = 440 ($M^+$+1)

| Elemental Analysis: $C_{25}H_{29}NO_6 \cdot 0.3H_2O$ | | | |
|---|---|---|---|
| Calcd.(%) | C, 67.49; | H, 6.71; | N, 3.15 |
| Found (%) | C, 67.55; | H, 6.93; | N, 3.15 |

Example 21

(Z)-2-(2,3-Dihydroxypropylthio)-3-(indol-3-yl)-1-(3,4,5-trimethoxyphenyl)-2-propen-1-one (Compound 21)

2-(2,3-Dihydroxypropylthio)-3',4',5'-trimethoxyacetophenone (3.16 g) obtained in Reference Example 16 and indole-3-carbaldehyde (1.45 g) were dissolved in ethanol (50 ml), and piperidine (0.85 g) was added thereto, followed by heating under reflux for 72 hours. The reaction solution was concentrated under reduced pressure and the residue was purified by silica gel column chromatography. The obtained crude crystals were recrystallized from a mixed solvent

of ethanol and water (1:4) to give Compound 21 (2.04 g).

$^1$H-NMR (270 MHz, $CDCl_3$) δ 2.31 (t, J = 5.9 Hz, 1H), 2.83 (dd, J = 13.9, 8.9 Hz, 1H), 3.09 (dd, J = 13.9, 4.0 Hz, 1H), 3.58 (dt, J = 11.2, 5.9 Hz, 1H), 3.69 (m, 1H), 3.79 (m, 1H), 3.88 (s, 6H), 3.97 (s, 3H), 4.11 (d, J = 3.5 Hz, 1H), 7.11 (s, 2H), 7.21 (m, 1H), 7.29 (m, 1H), 7.46 (brd, J = 7.9 Hz, 1H), 7.54 (brd, J = 7.4 Hz, 1H), 7.97 (s, 1H), 8.61 (d, J = 3.0 Hz, 1H), 9.01 (s, 1H)

EI-MS m/z = 443 ($M^+$)

| Elemental Analysis: $C_{23}H_{25}NO_6S \cdot 0.9H_2O$ | | | |
|---|---|---|---|
| Calcd.(%) | C, 60.09; | H, 5.88; | N, 3.05 |
| Found (%) | C, 60.11; | H, 6.01; | N, 3.03 |

Example 22

(Z)-2-(2,3-Dihydroxypropylthio)-3-(1-methylindol-3-yl)-1-(3,4,5-trimethoxyphenyl)-2-propen-1-one (Compound 22)

2-(2,3-Dihydroxypropylthio)-3',4',5'-trimethoxyacetophenone (1.00 g) obtained in Reference Example 16 and 1-methylindole-3-carbaldehyde (0.50 g) were dissolved in ethanol (8 ml), and piperidine (0.31 g) was added thereto, followed by heating under reflux for 48 hours. The reaction solution was concentrated under reduced pressure and the residue was purified by silica gel column chromatography. The obtained crude crystals were recrystallized from a mixed solvent of ethanol and water (1:1) to give Compound 22 (844.8 mg).

$^1$H-NMR (270 MHz, $CDCl_3$) δ 2.24 (t, J = 6.3 Hz, 1H), 2.82 (dd, J = 13.6, 8.7 Hz, 1H), 3.10 (dd, J = 13.6, 3.5 Hz, 1H), 3.57 (m, 1H), 3.69 (m, 1H), 3.78 (m, 1H), 3.87 (s, 6H), 3.94 (s, 3H), 3.97 (s, 3H), 4.24 (d, J = 4.0 Hz, 1H), 7.08 (s, 2H), 7.22 (m, 1H), 7.33 (m, 1H), 7.39 (d, J = 7.9 Hz, 1H), 7.53 (d, J = 7.9 Hz, 1H), 8.00 (s, 1H), 8.52 (s, 1H)

FAB-MS m/z = 458 ($M^+$+1)

| Elemental Analysis: $C_{24}H_{27}NO_6S$ | | | |
|---|---|---|---|
| Calcd.(%) | C, 63.00; | H, 5.95; | N, 3.06 |
| Found (%) | C, 62.85; | H, 5.97; | N, 3.02 |

Example 23

(Z)-2-(2,3-Dihydroxypropylthio)-3-(4-methylindol-3-yl)-1-(3,4,5-trimethoxyphenyl)-2-propen-1-one (Compound 23)

2-(2,3-Dihydroxypropylthio)-3',4',5'-trimethoxyacetophenone (397.5 mg) obtained in Reference Example 16 and 4-methylindole-3-carbaldehyde (WO95/14003) (200.0 mg) were dissolved in ethanol (2 ml), and piperidine (107.3 mg) was added thereto, followed by heating under reflux for 48 hours. The reaction solution was concentrated under reduced pressure and the residue was purified by silica gel column chromatography. The obtained crude crystals were recrystallized from a mixed solvent of ethyl acetate and hexane (2:1) and purified by preparative HPLC (YMC pack ODS, SH-343-5, S-5, 120A, 250 x 20 mm, acetonitrile:water = 40:60). The eluate was concentrated under reduced pressure and the residue was recrystallized from isopropyl ether to give Compound 23 (249.0 mg).

$^1$H-NMR (270 MHz, $CDCl_3$) δ 2.38 (t, J = 5.8 Hz, 1H), 2.41 (s, 3H), 2.83 (dd, J = 13.9, 8.6 Hz, 1H), 3.08 (dd, J = 13.9, 4.0 Hz, 1H), 3.57 (dt, J = 11.3, 5.8 Hz, 1H), 3.70 (m, 1H), 3.81 (m, 1H), 3.88 (s, 6H), 3.94 (s, 3H), 4.13 (d, J = 3.3 Hz, 1H), 6.93 (d, J = 7.6 Hz, 1H), 7.05 (s, 2H), 7.14 (t, J = 7.6 Hz, 1H), 7.30 (d, J = 7.6 Hz, 1H), 8.32 (s, 1H), 8.69 (d, J = 3.0 Hz, 1H), 9.07 (s, 1H)

FAB-MS m/z = 458 ($M^+$+1)

| Elemental Analysis: $C_{24}H_{27}NO_6S$ | | | |
|---|---|---|---|
| Calcd.(%) | C, 63.00; | H, 5.95; | N, 3.06 |
| Found (%) | C, 63.00; | H, 5.96; | N, 3.05 |

Example 24

(Z)-3-(4-Chloroindol-3-yl)-2-(2,3-dihydroxypropylthio)-1-(3,4,5-trimethoxyphenyl)-2-propen-1-one (Compound 24)

2-(2,3-Dihydroxypropylthio)-3',4',5'-trimethoxyacetophenone (948.0 mg) obtained in Reference Example 16

and 4-chloroindole-3-carbaldehyde [Can. J. Chem., 41, 1585 (1963)] (537.0 mg) were dissolved in ethanol (6 ml), and piperidine (296.7 mg) was added thereto, followed by heating under reflux for 48 hours. The reaction solution was concentrated under reduced pressure and the residue was purified by silica gel column chromatography. The obtained crude crystals were recrystallized from a mixed solvent of ethyl acetate and hexane (5:3) to give Compound 24 (909.9 mg).

$^1$H-NMR (270 MHz, $CDCl_3$) δ 2.48 (brs, 1H), 2.87 (dd, J = 13.6, 8.4 Hz, 1H), 3.08 (dd, J = 13.6, 4.0 Hz, 1H), 3.58 (m, 1H), 3.72 (m, 1H), 3.83 (m, 1H), 3.91 (s, 6H), 3.94 (s, 3H), 4.00 (d, J = 3.6 Hz, 1H), 7.11 (s, 2H), 7.15-7.17 (m, 2H), 7.38 (m, 1H), 8.64 (d, J = 2.6 Hz, 1H), 8.77 (s, 1H), 9.37 (s, 1H)

FAB-MS m/z = 478, 480 ($M^+$+1)

| Elemental Analysis: $C_{23}H_{24}ClNO_6S$ | | | |
|---|---|---|---|
| Calcd.(%) | C, 57.80; | H, 5.06; | N, 2.93 |
| Found (%) | C, 58.08; | H, 5.16; | N, 2.86 |

Example 25

(Z)-2-(2,3-Dihydroxypropylthio)-3-(5-methylindol-3-yl)-1-(3,4,5-trimethoxyphenyl)-2-propen-1-one (Compound 25)

2-(2,3-Dihydroxypropylthio)-3',4',5'-trimethoxyacetophenone (948.0 mg) obtained in Reference Example 16 and 5-methylindole-3-carbaldehyde (WO95/14003) (477.0 mg) were dissolved in ethanol (6 ml), and piperidine (255.5 mg) was added thereto, followed by heating under reflux for 36 hours. The reaction solution was concentrated under reduced pressure and the residue was purified by silica gel column chromatography. The obtained crude crystals were recrystallized from a mixed solvent of ethanol and water (2:3) to give Compound 25 (710.9 mg).

$^1$H-NMR (270 MHz, $CDCl_3$) δ 2.32 (t, J = 5.9 Hz, 1H), 2.43 (s, 3H), 2.81 (dd, J = 13.7, 8.6 Hz, 1H), 3.08 (dd, J = 13.7, 3.8 Hz, 1H), 3.56 (m, 1H), 3.68 (m, 1H), 3.78 (m, 1H), 3.89 (s, 6H), 3.97 (s, 3H), 4.15 (d, J = 3.6 Hz, 1H), 7.11 (m, 1H), 7.12 (s, 2H), 7.30 (brs, 1H), 7.34 (d, J = 8.3 Hz, 1H), 7.95 (brs, 1H) , 8.58 (d, J = 3.0 Hz, 1H) , 8.90 (s, 1H)

FAB-MS m/z = 458 ($M^+$+1)

| Elemental Analysis: $C_{24}H_{27}NO_6S$ | | | |
|---|---|---|---|
| Calcd.(%): | C, 63.00; | H, 5.95; | N, 3.06 |
| Found (%): | C, 63.33; | H, 6.10; | N, 3.13 |

Example 26

(Z)-3-(5-Chloroindol-3-yl)-2-(2,3-dihydroxypropylthio)-1-(3,4,5-trimethoxyphenyl)-2-propen-1-one (Compound 26)

2-(2,3-Dihydroxypropylthio)-3',4',5'-trimethoxyacetophenone (948.0 mg) obtained in Reference Example 16 and 5-chloroindole-3-carbaldehyde (WO95/14003) (537.0 mg) were dissolved in ethanol (6 ml), and piperidine (296.7 mg) was added thereto, followed by heating under reflux for 48 hours. The reaction solution was concentrated under reduced pressure and the residue was purified by silica gel column chromatography. The obtained crude crystals were recrystallized from ethyl acetate to give Compound 26 (837.2 mg).

$^1$H-NMR (270 MHz, DMSO-$d_6$) δ 2.78 (dd, J = 13.2, 7.3 Hz, 1H), 2.90 (dd, J = 13.2, 4.6 Hz, 1H), 3.29-3.39 (m, 2H), 3.55 (m, 1H), 3.80 (s, 3H), 3.82 (s, 6H), 4.55 (t, J = 5.6 Hz, 1H), 4.86 (d, J = 5.3 Hz, 1H), 7.13 (s, 2H), 7.18 (dd, J = 8.8, 2.0 Hz, 1H), 7.50 (d, J = 8.8 Hz, 1H), 7.54 (s, 1H), 7.59 (d, J = 2.0 Hz, 1H), 8.45 (s, 1H), 12.00 (brs, 1H)

FAB-MS m/z = 478, 480 ($M^+$+1)

| Elemental Analysis: $C_{23}H_{24}ClNO_6S$ | | | |
|---|---|---|---|
| Calcd.(%) | C, 57.80; | H, 5.06; | N, 2.93 |
| Found (%) | C, 57.86; | H, 5.13; | N, 2.76 |

Example 27

(Z)-2-(2,3-Dihydroxypropylthio)-3-(5-fluoroindol-3-yl)-1-(3,4,5-trimethoxyphenyl)-2-propen-1-one (Compound 27)

2-(2,3-Dihydroxypropylthio)-3',4',5'-trimethoxyacetophenone (948.0 mg) obtained in Reference Example 16

and 5-fluoroindole-3-carbaldehyde (WO95/14003) (489.0 mg) were dissolved in ethanol (20 ml), and piperidine (85.0 mg) was added thereto, followed by heating under reflux for 36 hours. The reaction solution was concentrated under reduced pressure and the residue was purified by silica gel column chromatography. The obtained crude crystals were recrystallized from a mixed solvent of ethanol and water (1:1) to give Compound 27 (618.1 mg).

$^1$H-NMR (270 MHz, DMSO-$d_6$) δ 2.78 (dd, J = 13.1, 7.2 Hz, 1H), 2.90 (dd, J = 13.1, 4.7 Hz, 1H), 3.25-3.40 (m, 2H), 3.54 (m, 1H), 3.79 (s, 3H), 3.81 (s, 6H), 4.56 (t, J = 5.7 Hz, 1H), 4.87 (d, J = 5.4 Hz, 1H), 7.02 (td, J = 9.0, 2.5 Hz, 1H), 7.11 (s, 2H), 7.31 (dd, J = 9.9, 2.5 Hz, 1H), 7.48 (dd, J = 9.0, 4.0 Hz, 1H), 7.53 (s, 1H), 8.48 (s, 1H), 11.96 (brs, 1H)

EI-MS m/z = 461 ($M^+$)

| Elemental Analysis: $C_{23}H_{24}FNO_6S \cdot 0.9H_2O$ | | | |
|---|---|---|---|
| Calcd.(%) | C, 57.83; | H, 5.44; | N, 2.93 |
| Found (%) | C, 57.89; | H, 5.53; | N, 2.93 |

Example 28

(Z)-2-(2,3-Dihydroxypropylthio)-3-(6-methylindol-3-yl)-1-(3,4,5-trimethoxyphenyl)-2-propen-1-one (Compound 28)

2-(2,3-Dihydroxypropylthio)-3',4',5'-trimethoxyacetophenone (1.58 g) obtained in Reference Example 16 and 6-methylindole-3-carbaldehyde (975.0 mg) were dissolved in ethanol (10 ml), and piperidine (494.5 mg) was added thereto, followed by heating under reflux for 36 hours. The reaction solution was concentrated under reduced pressure and the residue was purified by silica gel column chromatography. The obtained crude crystals were recrystallized from a mixed solvent of ethyl acetate and hexane (5:3) to give Compound 28 (1.23 g).

$^1$H-NMR (270 MHz, $CDCl_3$) δ 2.36 (s, 1H), 2.46 (s, 3H), 2.82 (dd, J = 13.9, 8.9 Hz, 1H), 3.08 (dd, J = 13.9, 4.0 Hz, 1H), 3.55 (m, 1H), 3.68 (m, 1H), 3.77 (m, 1H), 3.87 (s, 6H), 3.97 (s, 3H), 4.16 (s, 1H), 7.04 (d, J = 7.7 Hz, 1H), 7.10 (s, 2H), 7.24 (s, 1H), 7.41 (d, J = 7.7 Hz, 1H), 7.96 (s, 1H), 8.54 (d, J = 3.0 Hz, 1H), 8.96 (s, 1H)

EI-MS m/z = 457 ($M^+$)

| Elemental Analysis: $C_{24}H_{27}NO_6S \cdot 0.2H_2O$ | | | |
|---|---|---|---|
| Calcd.(%) | C, 62.51; | H, 5.99; | N, 3.04 |
| Found (%) | C, 62.46; | H, 6.11; | N, 2.95 |

Example 29

(Z)-2-(2,3-Dihydroxypropylthio)-3-(6-ethylindol-3-yl)-1-(3,4,5-trimethoxyphenyl)-2-propen-1-one (Compound 29)

2-(2,3-Dihydroxypropylthio)-3',4',5'-trimethoxyacetophenone (2.21 g) obtained in Reference Example 16 and 6-ethylindole-3-carbaldehyde (WO95/14003) (1.21 g) were dissolved in ethanol (14 ml), and piperidine (596.1 mg) was added thereto, followed by heating under reflux for 30 hours. The reaction solution was concentrated under reduced pressure and the residue was purified by silica gel column chromatography. The obtained crude crystals were recrystallized from a mixed solvent of ethanol and water (2:3) to give Compound 29 (1.48 g).

$^1$H-NMR (270 MHz, DMSO-$d_6$) δ 1.22 (t, J = 7.6 Hz, 3H), 2.70 (q, J = 7.6 Hz, 2H), 2.80 (dd, J = 13.3, 7.3 Hz, 1H), 2.91 (dd, J = 13.3, 4.6 Hz, 1H), 3.33 (m, 2H), 3.55 (m, 1H), 3.79 (s, 3H), 3.81 (s, 6H), 4.57 (t, J = 5.5 Hz, 1H), 4.87 (d, J = 5.5 Hz, 1H), 6.98 (d, J = 8.1 Hz, 1H), 7.10 (s, 2H), 7.28 (s, 1H), 7.38 (d, J = 8.1 Hz, 1H), 7.60 (s, 1H), 8.37 (d, J = 2.3 Hz, 1H), 11.79 (d, J = 2.3 Hz, 1H)

FAB-MS m/z = 472 ($M^+$+1)

| Elemental Analysis: $C_{25}H_{29}NO_6S$ | | | |
|---|---|---|---|
| Calcd.(%) | C, 63.68; | H, 6.20; | N, 2.97 |
| Found (%) | C, 63.79; | H, 6.34; | N, 2.93 |

Example 30

(Z)-2-(2,3-Dihydroxypropylthio)-3-(6-isopropylindol-3-yl)-1-(3,4,5-trimethoxyphenyl)-2-propen-1-one (Compound 30)

2-(2,3-Dihydroxypropylthio)-3',4',5'-trimethoxyacetophenone (1.58 g) obtained in Reference Example 16 and 6-isopropylindole-3-carbaldehyde (WO95/14003) (935.0 mg) were dissolved in ethanol (10 ml), and piperidine (425.8 mg) was added thereto, followed by heating under reflux for 24 hours. The reaction solution was concentrated under reduced pressure and the residue was purified by silica gel column chromatography. The obtained crude crystals were recrystallized from a mixed solvent of ethyl acetate and hexane (1:1) to give Compound 30 (1.23 g).

$^1$H-NMR (270 MHz, $CDCl_3$) δ 1.30 (d, J = 6.9 Hz, 6H), 2.33 (t, J = 6.1 Hz, 1H), 2.81 (dd, J = 13.5, 8.7 Hz, 1H), 3.03 (m, 1H), 3.08 (dd, J = 13.5, 3.6 Hz, 1H), 3.56 (m, 1H), 3.63-3.83 (m, 2H), 3.88 (s, 6H), 3.97 (s, 3H), 4.19 (d, J = 3.0 Hz, 1H), 7.10 (s, 2H), 7.13 (d, J = 8.3 Hz, 1H), 7.30 (s, 1H), 7.43 (d, J = 8.3 Hz, 1H), 7.97 (s, 1H), 8.57 (d, J = 3.0 Hz, 1H), 8.92 (brs, 1H)

FAB-MS m/z = 486 ($M^+$+1)

| Elemental Analysis: $C_{26}H_{31}NO_6S \cdot 0.6H_2O$ | | | |
|---|---|---|---|
| Calcd.(%) | C, 63.45; | H, 6.62; | N, 2.69 |
| Found (%) | C, 63.66; | H, 6.71; | N, 2.90 |

Example 31

(Z)-3-(6-Chloroindol-3-yl)-2-(2,3-dihydroxypropylthio)-1-(3,4,5-trimethoxyphenyl)-2-propen-1-one (Compound 31)

2-(2,3-Dihydroxypropylthio)-3',4',5'-trimethoxyacetophenone (948.0 mg) obtained in Reference Example 16 and 6-chloroindole-3-carbaldehyde (WO95/14003) (538.5 mg) were dissolved in ethanol (20 ml), and piperidine (255.0 mg) was added thereto, followed by heating under reflux for 38 hours. The reaction solution was concentrated under reduced pressure and the residue was purified by silica gel column chromatography. The obtained crude crystals were recrystallized from a mixed solvent of ethyl acetate and hexane (1:1) to give Compound 31 (270.3 mg).

$^1$H-NMR (270 MHz, $CDCl_3$) δ 2.51 (s, 1H), 2.86 (dd, J = 13.9, 8.4 Hz, 1H), 3.06 (dd, J = 13.9, 4.0 Hz, 1H), 3.56 (m, 1H), 3.68 (m, 1H), 3.78 (m, 1H), 3.88 (s, 6H), 3.97 (s, 3H), 4.04 (s, 1H), 7.11 (s, 2H), 7.16 (dd, J = 8.9, 1.8 Hz, 1H), 7.43 (d, J = 8.9 Hz, 1H), 7.46 (d, J = 1.8 Hz, 1H), 7.83 (s, 1H), 8.57 (d, J = 3.0 Hz, 1H), 9.25 (s, 1H)

FAB-MS m/z = 478, 480 ($M^+$+1)

| Elemental Analysis: $C_{23}H_{24}ClNO_6S \cdot 0.9H_2O$ | | | |
|---|---|---|---|
| Calcd.(%) | C, 57.83; | H, 5.44; | N, 2.93 |
| Found (%) | C, 57.89; | H, 5.53; | N, 2.93 |

Example 32

(Z)-2-(2,3-Dihydroxypropylthio)-3-(6-fluoroindol-3-yl)-1-(3,4,5-trimethoxyphenyl)-2-propen-1-one (Compound 32)

2-(2,3-Dihydroxypropylthio)-3',4',5'-trimethoxyacetophenone (1.62 g) obtained in Reference Example 16 and 6-fluoroindole-3-carbaldehyde [J. Med. Chem., 6, 716 (1963)] (838.2 mg) were dissolved in ethanol (10 ml), and piperidine (437.7 mg) was added thereto, followed by heating under reflux for 36 hours. The reaction solution was concentrated under reduced pressure and the residue was purified by silica gel column chromatography. The obtained crude crystals were recrystallized from ethanol to give Compound 32 (638.8 mg).

$^1$H-NMR (270 MHz, $CDCl_3$) δ 2.34 (t, J = 6.3 Hz, 1H), 2.84 (dd, J = 13.8, 8.6 Hz, 1H), 3.08 (dd, J = 13.8, 3.8 Hz, 1H), 3.58 (m, 1H), 3.69 (m, 1H), 3.79 (m, 1H), 3.88 (s, 6H), 3.97 (s, 3H), 3.99 (d, J = 4.0 Hz, 1H), 6.97 (td, J = 8.9, 2.2 Hz, 1H), 7.11 (s, 2H), 7.15 (dd, J = 9.1, 2.2 Hz, 1H), 7.45 (dd, J = 8.9, 5.0 Hz, 1H), 7.84 (s, 1H), 8.55 (d, J = 2.6 Hz, 1H), 9.10 (brs, 1H)

FAB-MS m/z = 462 ($M^+$+1)

| Elemental Analysis: $C_{23}H_{24}FNO_6S$ | | | |
|---|---|---|---|
| Calcd.(%) | C, 59.86; | H, 5.24; | N, 3.04 |
| Found (%) | C, 59.68; | H, 5.17; | N, 2.89 |

Example 33

(Z)-3-(6-Acetamidoindol-3-yl)-2-(2,3-dihydroxypropylthio)-1-(3,4,5-trimethoxyphenyl)-2-propen-1-one (Compound 33)

2-(2,3-Dihydroxypropylthio)-3',4',5'-trimethoxyacetophenone (1.58 g) obtained in Reference Example 16 and 6-acetoamidoindole-3-carbaldehyde (WO95/14003) (1.64 g) were dissolved in ethanol (20 ml), and piperidine (690.0 mg) was added thereto, followed by heating under reflux for 38 hours. The precipitated crystals were collected by filtration and the obtained crude crystals were recrystallized from a mixed solvent of ethanol, methanol, N,N-dimethylformamide, and water to give Compound 33 (707.3 mg).

$^1$H-NMR (270 MHz, DMSO-$d_6$) $\delta$ 2.05 (s, 3H), 2.79 (dd, J = 13.4, 6.9 Hz, 1H), 2.91 (dd, J = 13.4, 5.0 Hz, 1H), 3.27-3.38 (m, 2H), 3.54 (m, 1H), 3.79 (s, 3H), 3.81 (s, 6H), 4.55 (t, J = 5.7 Hz, 1H), 4.86 (d, J = 5.0 Hz, 1H), 7.09 (s, 2H), 7.10 (dd, J = 8.9, 1.5 Hz, 1H), 7.38 (d, J = 8.9 Hz, 1H), 7.57 (s, 1H), 8.07 (d, J = 1.5 Hz, 1H), 8.36 (s, 1H), 9.92 (s, 1H), 11.80 (s, 1H)

EI-MS m/z = 500 ($M^+$)

| Elemental Analysis: $C_{25}H_{28}N_2O_7S$ | | | |
|---|---|---|---|
| Calcd.(%) | C, 59.99; | H, 5.64; | N, 5.60 |
| Found (%) | C, 59.97; | H, 5.74; | N, 5.72 |

Example 34

(Z)-3-(6-Acetamido-1-methylindol-3-yl)-2-(2,3-dihydroxypropylthio)-1-(3,4,5-trimethoxyphenyl)-2-propen-1-one (Compound 34)

Compound 33 (300.0 mg) obtained in Example 33 was dissolved in a mixed solvent of N,N-dimethylformamide and tetrahydrofuran (1:1), and methyl iodide (93.6 mg) and then sodium hydride (26.4 mg, 60% mineral oil dispersion) were added thereto, followed by stirring for 1.5 hours. The reaction solution was subjected to partitioning between chloroform and water, and the organic layer was successively washed with water and a saturated saline, dried over anhydrous magnesium sulfate, and concentrated under reduced pressure. The obtained crude crystals were washed with chloroform and recrystallized from a mixed solvent of ethanol, N,N-dimethylformamide, and water to give Compound 34 (249.7 mg).

$^1$H-NMR (270 MHz, DMSO-$d_6$) $\delta$ 2.06 (s, 3H), 2.80 (dd, J = 13.2, 7.3 Hz, 1H), 2.91 (dd, J = 13.2, 4.6 Hz, 1H), 3.27-3.38 (m, 2H), 3.53 (m, 1H), 3.79 (s, 3H), 3.80 (s, 6H), 3.86 (s, 3H), 4.54 (brs, 1H), 4.85 (d, J = 4.6 Hz, 1H), 7.09 (s, 2H), 7.14 (d, J = 8.8 Hz, 1H), 7.40 (d, J = 8.8 Hz, 1H), 7.54 (s, 1H), 8.01 (s, 1H), 8.37 (s, 1H), 9.92 (s, 1H)

EI-MS m/z = 514 ($M^+$)

| Elemental Analysis: $C_{26}H_{30}N_2O_7S$ | | | |
|---|---|---|---|
| Calcd.(%) | C, 60.69; | H, 5.88; | N, 5.44 |
| Found (%) | C, 60.73; | H, 5.99; | N, 5.29 |

Example 35

3-(Indol-3-yl)-2-[(2S),(3R)-2,3,4-trihydroxybutylthio]-1-(3,4,5-trimethoxyphenyl)-2-propen-1-one (Compound 35)

3',4',5'-Trimethoxy-2-[(2S),(3R)-2,3,4-trihydroxybutylthio]acetophenone (268.0 mg) obtained in Reference Example 22 and indole-3-carbaldehyde (112.3 mg) were dissolved in ethanol (4 ml), and piperidine (66.0 mg) was added thereto, followed by heating under reflux for 96 hours. The reaction solution was concentrated under reduced pressure and the residue was purified by silica gel column chromatography. The obtained crude crystals were recrystallized from a mixed solvent of ethyl acetate and hexane to give Compound 35 (56.9 mg).

$^1$H-NMR (270 MHz, $CDCl_3$) $\delta$ 2.96 (dd, J = 13.6, 8.7 Hz, 1H), 3.09 (dd, J = 13.6, 4.0 Hz, 1H), 3.23 (brs, 1H), 3.52-3.82 (m, 5H), 3.84 (s, 6H), 3.96 (s, 3H), 4.81 (d, J = 2.0 Hz, 1H), 7.08 (s, 2H), 7.17 (m, 1H), 7.24 (m, 1H), 7.42 (d, J = 7.6 Hz, 1H), 7.49 (d. J = 7.6 Hz, 1H), 7.98 (s, 1H), 8.65 (d, J = 2.6 Hz, 1H), 9.56 (brs, 1H)

FAB-MS m/z = 474 ($M^+$+1)

| Elemental Analysis: $C_{24}H_{27}NO_7S \cdot 0.6H_2O$ | | | |
|---|---|---|---|
| Calcd.(%) | C, 59.52; | H, 5.88; | N, 2.89 |
| Found (%) | C, 59.60; | H, 5.96; | N, 2.86 |

Example 36

3-(6-Methylindol-3-yl)-2-[(2S),(3R)-2,3,4-trihydroxybutylthio]-1-(3,4,5-trimethoxyphenyl)-2-propen-1-one (Compound 36)

3',4',5'-Trimethoxy-2-[(2S),(3R)-2,3,4-trihydroxybutylthio]acetophenone (1.73 g) obtained in Reference Example 22 and 6-methylindole-3-carbaldehyde (0.80 g) were dissolved in ethanol (10 ml), and piperidine (0.43g) was added thereto, followed by heating under reflux for 26 hours. The reaction solution was concentrated under reduced pressure and the residue was purified by silica gel column chromatography. The obtained crude crystals were recrystallized from a mixed solvent of ethyl acetate and hexane to give Compound 36 (1.22 g).

$^1$H-NMR (270 MHz, $CDCl_3$) δ 2.43 (s, 3H), 2.96 (dd, J = 13.6, 8.7 Hz, 1H), 3.10 (dd, J = 13.6, 4.0 Hz, 1H), 3.23 (brs, 1H), 3.53-3.82 (m, 5H), 3.84 (s, 6H), 3.96 (s, 3H), 4.84 (brs, 1H), 7.00 (d, J = 8.2 Hz, 1H), 7.07 (s, 2H), 7.20 (s, 1H), 7.36 (d, J = 8.2 Hz, 1H), 7.97 (s, 1H), 8.59 (d, J = 2.6 Hz, 1H), 9.39 (brs, 1H)

FAB-MS m/z = 488 ($M^+$+1)

| Elemental Analysis: $C_{25}H_{29}NO_7S$ | | | |
|---|---|---|---|
| Calcd.(%) | C, 61.59; | H, 6.00; | N, 2.87 |
| Found (%) | C, 61.26; | H, 6.12; | N, 2.82 |

Example 37

3-(6-Methylindol-3-yl)-2-[(2R),(3S)-2,3,4-trihydroxybutylthio)-1-(3,4,5-trimethoxyphenyl)-2-propen-1-one (Compound 37)

3',4',5'-Trimethoxy-2-[(2R),(3S)-2,3,4-trihydroxybutylthio]acetophenone (1.73 g) obtained in Reference Example 23 and 6-methylindole-3-carbaldehyde (0.80 g) were dissolved in ethanol (10 ml), and piperidine (0.43g) was added thereto, followed by heating under reflux for 48 hours. The reaction solution was concentrated under reduced pressure and the residue was purified by silica gel column chromatography. The obtained crude crystals were recrystallized from a mixed solvent of ethyl acetate and hexane to give Compound 37 (1.12 g).

$^1$H-NMR (270 MHz, $CDCl_3$) δ 2.43 (s, 3H), 2.95 (dd, J = 13.6, 8.7 Hz, 1H), 3.10 (dd, J = 13.6, 4.0 Hz, 1H), 3.17 (brs, 1H), 3.53 (d, J = 6.3 Hz, 1H), 3.64-3.82 (m, 4H), 3.84 (s, 6H), 3.96 (s, 3H), 4.83 (d, J = 4.0 Hz, 1H), 7.01 (d, J = 8.2 Hz, 1H), 7.07 (s, 2H), 7.20 (s, 1H), 7.37 (d, J = 8.2 Hz, 1H), 7.97 (s, 1H), 8.59 (d, J = 2.6 Hz, 1H), 9.36 (brs, 1H)

FAB-MS m/z = 488 ($M^+$+1)

| Elemental Analysis: $C_{25}H_{29}NO_7S$ | | | |
|---|---|---|---|
| Calcd.(%) | C, 61.59; | H, 6.00; | N, 2.87 |
| Found (%) | C, 61.56; | H, 6.14; | N, 2.82 |

Example 38

2-(2,3-Dihydroxypropylthio)-3-(indol-5-yl)-1-(3,4,5-trimethoxyphenyl)-2-propen-1-one (Compound 38a, Compound 38b)

2-(2,3-Dihydroxypropylthio)-3',4',5'-trimethoxyacetophenone (316.0 mg) obtained in Reference Example 16 and indole-5-carbaldehyde (145.0 mg) obtained in Reference Example 24 were dissolved in ethanol (5 ml), and piperidine (850.0 mg) was added thereto, followed by heating under reflux for 72 hours. The reaction solution was concentrated under reduced pressure and the residue was purified by silica gel column chromatography. The obtained mixture was purified by preparative HPLC (YMC pack ODS, SH-343-5, S-5, 120A, 250 x 20 mm, acetonitrile:water = 35:65) and the eluates were concentrated under reduced pressure to give Compound 38a (36.4 mg, Z form) and Compound 38b (17.0 mg, E form).

Compound 38a:

$^1$H-NMR (270 MHz, $CDCl_3$) δ 2.01 (brs, 1H), 2.62 (brs, 1H), 2.82 (dd, J = 13.8, 8.4 Hz, 1H), 2.99 (dd, J = 13.8, 4.0 Hz, 1H), 3.54 (dd, J = 11.8, 5.8 Hz, 1H), 3.66 (dd, J = 11.4, 3.5 Hz, 1H), 3.78 (m, 1H), 3.92 (s, 6H), 3.99 (s, 3H), 6.64 (brs, 1H), 7.21 (s, 2H), 7.28-7.31 (m, 2H), 7.46 (brd, J = 8.6 Hz, 1H), 7.84 (dd, J = 8.6, 1.3 Hz, 1H), 8.25 (s, 1H), 8.85 (s, 1H)

FAB-MS m/z = 444 ($M^+$+1)

Compound 38b:

$^1$H-NMR (270 MHz, $CDCl_3$) δ 2.66 (brs, 1H), 2.81 (dd, J = 14.3, 6.9 Hz, 1H), 2.89 (dd, J = 14.3, 5.5 Hz, 1H), 3.23 (brs, 1H), 3.67-3.98 (m, 3H), 3.83 (s, 6H), 3.90 (s, 3H), 6.47 (brs, 1H), 7.03 (dd, J = 8.6, 1.3 Hz, 1H), 7.17-7.22 (m, 2H), 7.28 (s, 2H), 7.45 (s, 1H), 7.52 (s, 1H), 8.39 (s, 1H)

FAB-MS m/z = 444 ($M^+$+1)

Industrial Applicability

According to the present invention, there can be provided propenone derivatives having an excellent antitumor activity.

## Claims

**1.** A propenone derivative represented by the following formula (I):

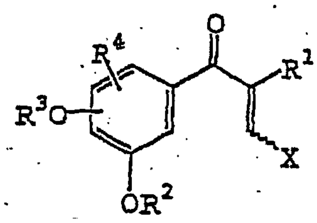

(I)

wherein $R^1$ represents $C_1$-$C_6$ alkyl substituted with 1 to 4 hydroxy groups, or $YR^5$ (wherein Y represents S or O, and $R^5$ represents substituted or unsubstituted $C_1$-$C_6$alkyl, substituted or unsubstituted aryl, substituted or unsubstituted heteroaryl, or a substituted or unsubstituted cyclic ether residue), $R^2$ and $R^3$ represent independently hydrogen, $C_1$-$C_6$ alkyl, or substituted or unsubstituted aralkyl, or $R^2$ and $R^3$ are combined to represent substituted or unsubstituted methylene or ethylene, $R^4$ represents hydrogen, hydroxy, $C_1$-$C_6$ alkyl, substituted or unsubstituted aralkyl, $C_1$-$C_6$ alkoxy, substituted or unsubstituted aralkyloxy, or halogen, and X represents substituted or unsubstituted indolyl;

wherein the substituted $C_1$-$C_6$ alkyl has the same or different 1 to 4 substituents selected from the group consisting of vinyl, hydroxy, $C_1$-$C_6$alkoxy, aryloxy, amino, $C_1$-$C_6$alkylamino, di($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl, tri ($C_1$-$C_6$ alkyl)silyl and a cyclic ether residue;

the substituted aryl, substituted heteroaryl, substituted cyclic ether residue, substituted aralkyl, and substituted aralkyloxy each has the same or different 1 to 4 substituents selected from the group consisting of $C_1$-$C_6$ alkyl, vinyl, hydroxy, hydroxymethyl, $C_1$-$C_6$ alkoxy, aryloxy, amino, $C_1$-$C_6$ alkylamino, di($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl, tri($C_1$-$C_6$ alkyl)silyl, aralkyl and a cyclic ether residue;

the substituted methylene and substituted ethylene each has the same or different 1 to 3 $C_1$-$C_6$ alkyl;

the substituted indolyl has on the nitrogen atom at position 1 the substituent (s) selected from the group consisting of $C_1$-$C_6$ alkyl, $C_2$-$C_7$ alkanoyl, $C_1$-$C_6$ alkoxycarbonyl and aralkyl, and on the carbon atoms at positions 2 to 7 the substituent(s) selected from the group consisting of $C_1$-$C_6$ alkyl, $C_1$-$C_6$ alkoxy, amino, $C_1$-$C_6$ alkylamino, di($C_1$-$C_6$ alkyl)amino, $C_2$-$C_7$ alkanoylamino, $C_1$-$C_6$ alkoxycarbonylamino, halogen, nitro, carboxy, $C_2$-$C_7$ alkanoyl, $C_1$-$C_6$ alkoxycarbonyl and aralkyl; the aryl and the aryl moiety of the aryloxy represent phenyl or naphthyl;

the heteroaryl represents pyridyl, pyrazinyl, pyrimidinyl, pyridazinyl, quinolinyl, isoquinolinyl, phthalazinyl,

quinazolinyl, quinoxalinyl, naphthyridinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl or purinyl;
the cyclic ether residue represents a cyclic ether residue having 2 to 6 carbon atoms; and
the aralkyl and the aralkyl moiety of the aralkyloxy represent an aralkyl group having 7 to 15 carbon atoms ;

or a pharmaceutically acceptable salt thereof.

**2.** A propenone derivative or a pharmaceutically acceptable salt thereof according to Claim 1, wherein $R^1$ represents $YR^5$ (wherein Y and $R^5$ have the same meanings as defined in claim 1).

**3.** A propenone derivative or a pharmaceutically acceptable salt thereof according to Claim 1, wherein $R^1$ represents. $C_1$-$C_6$ alkyl substituted with 1 to 4 hydroxy groups,

**4.** A propenone derivative or a pharmaceutically acceptable salt thereof according to any of Claims 1 to 3, wherein X represents substituted or unsubstituted indol-3-yl.

**5.** Pharmaceutical composition comprising a propenone derivative or a pharmaceutically acceptable salt thereof according to any of Claims 1 to 4.

**6.** Use of a propenone derivative or a pharmaceutically acceptable salt thereof as defined in any of Claims 1 to 4 for the preparation of a pharmaceutical composition for treating tumors,

**7.** Use of a propenone derivative or a pharmaceutically acceptable salt thereof as defined in any of Claims 1 to 4 for the preparation of a pharmaceutical composition having immunosuppressive activity.

**Patentansprüche**

**1.** Propenon-Derivat der folgenden Formel (I):

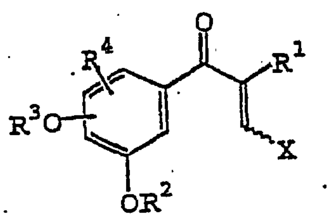

(I)

wobei $R^1$ einen mit 1 bis 4 Hydroxygruppen substituierten $C_1$-$C_6$-Alkylrest oder $YR^5$ bedeutet (wobei Y für S oder O steht, und $R^5$ einen substituierten oder unsubstituierten $C_1$-$C_6$-Alkylrest, einen substituierten oder unsubstituierten Arylrest, einen substituierten oder unsubstituierten Heteroarylrest, oder einen substituierten oder unsubstituierten cyclischen Etherrest bedeutet); $R^2$ und $R^3$ unabhängig ein Wasserstoffatom, einen $C_1$-$C_6$-Alkylrest oder einen substituierten oder unsubstituierten Aralkylrest bedeuten, oder $R^2$ und $R^3$ zusammengenommen eine substituierte oder unsubstituierte Methylen- oder Ethylengruppe bedeuten; $R^4$ ein Wasserstoffatom, eine Hydroxygruppe, einen $C_1$-$C_6$-Alkylrest, einen substituierten oder unsubstituierten Aralkylrest, einen $C_1$-$C_6$-Alkoxyrest, einen substituierten oder unsubstituierten Aralkyloxyrest oder ein Halogenatom bedeutet; und X eine substituierte oder unsubstituierte Indolylgruppe bedeutet;
wobei der substituierte $C_1$-$C_6$-Alkylrest 1 bis 4 gleiche oder verschiedene Substituenten aufweist, die ausgewählt sind aus Vinyl, Hydroxy, $C_1$-$C_6$-Alkoxy, Aryloxy, Amino, $C_1$-$C_6$-Alkylamino, Di($C_1$-$C_6$-alkyl)amino, $C_2$-$C_7$-Alkanoylamino, $C_1$-$C_6$-Alkoxycarbonylamino, Halogen, Nitro, Carboxy, $C_2$-$C_7$-Alkanoyl, $C_1$-$C_6$-Alkoxycarbonyl, Tri($C_1$-$C_6$-alkyl)silyl und einem cyclischen Etherrest;
der substituierte Aryl-, der substituierte Heteroaryl-, der substituierte cyclische Etherrest, der substituierte Aralkylrest und der substituierte Aralkyloxyrest jeweils 1 bis 4 gleiche oder verschiedene Substituenten aufweisen, die ausgewählt sind aus $C_1$-$C_6$-Alkyl, Vinyl, Hydroxy, Hydroxymethyl, $C_1$-$C_6$-Alkoxy, Aryloxy, Amino, $C_1$-$C_6$-Alkylamino, Di($C_1$-$C_6$-alkyl)amino, $C_2$-$C_7$-Alkanoylamino, $C_1$-$C_6$-Alkoxycarbonylamino, Halogen, Nitro, Carboxy, $C_2$-$C_7$-Alkanoyl, $C_1$-$C_6$-Alkoxycarbonyl, Tri($C_1$-$C_6$-alkyl)silyl, Aralkyl und einem cyclischen Etherrest; die substituierte Methylengruppe und die substituierte Ethylengruppe jeweils 1 bis 3 gleiche oder verschiedene

$C_1$-$C_6$-Alkylreste aufweisen;
die substituierte Indolylgruppe an dem Stickstoffatom an Position 1 den/die Substituent(en) aufweist, der/die ausgewählt ist/sind aus $C_1$-$C_6$-Alkyl, $C_2$-$C_7$-Alkanoyl, $C_1$-$C_6$-Alkoxycarbonyl und Aralkyl, und an den Kohlenstoffatomen an den Positionen 2 bis 7 den/die Substituent(en) aufweist, der/die ausgewählt ist/sind aus $C_1$-$C_6$-Alkyl, $C_1$-$C_6$-Alkoxy, Amino, $C_1$-$C_6$-Alkylamino, Di($C_1$-$C_6$-alkyl)amino, $C_2$-$C_7$-Alkanoylamino, $C_1$-$C_6$-Alkoxycarbonylamino, Halogen, Nitro, Carboxy, $C_2$-$C_7$-Alkanoyl, $C_1$-$C_6$-Alkoxycarbonyl und Aralkyl;
der Arylrest und die Aryleinheit des Aryloxyrests Phenyl oder Naphthyl darstellen; der Heteroarylrest Pyridyl, Pyrazinyl, Pyrimidinyl, Pyridazinyl, Chinolinyl, Isochinolinyl, Phthalazinyl, Chinazolinyl, Chinoxalinyl, Naphthyridinyl, Pyrrolyl, Pyrazolyl, Imidazolyl, Triazolyl, Tetrazolyl, Thienyl, Furyl, Thiazolyl, Oxazolyl, Indolyl, Indazolyl, Benzimidazolyl, Benzotriazolyl oder Purinyl darstellt;
der cyclische Etherrest einen cyclischen Etherrest mit 2 bis 6 Kohlenstoffatomen darstellt; und
der Aralkylrest und die Aralkyleinheit des Aralkyloxyrests einen Aralkylrest mit 7 bis 15 Kohlenstoffatomen darstellen;

oder ein pharmazeutisch verträgliches Salz davon.

**2.** Propenon-Derivat oder ein pharmazeutisch verträgliches Salz davon nach Anspruch 1, wobei $R^1$ für $YR^5$ steht (wobei Y und $R^5$ die gleichen Bedeutungen wie in Anspruch 1 definiert haben).

**3.** Propenon-Derivat oder ein pharmazeutisch verträgliches Salz davon nach Anspruch 1, wobei $R^1$ einen mit 1, bis 4 Hydroxygruppen substituierten $C_1$-$C_6$-Alkylrest bedeutet.

**4.** Propenon-Derivat oder ein pharmazeutisch verträgliches Salz davon nach einem der Ansprüche 1 bis 3, wobei X eine substituierte oder unsubstituierte Indol-3-yl-Gruppe bedeutet.

**5.** Arzneimittel, umfassend ein Propenon-Derivat oder ein pharmazeutisch verträgliches Salz davon nach einem der Ansprüche 1 bis 4.

**6.** Verwendung eines Propenon-Derivats oder eines pharmazeutisch verträglichen Salzes davon, wie in einem der Ansprüche 1 bis 4 definiert, zur Herstellung eines Arzneimittels zur Behandlung von Tumoren.

**7.** Verwendung eines Propenon-Derivats oder eines pharmazeutisch verträglichen Salzes davon, wie in einem der Ansprüche 1 bis 4 definiert, zur Herstellung eines Arzneimittels mit immunsuppressiver-Wirksamkeit.

**Revendications**

**1.** Dérivé de propénone, représenté par la formule (I) suivante :

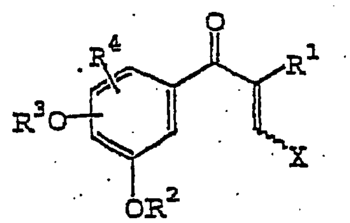

(I)

dans laquelle

$R^1$ représente un groupe alkyle en $C_{1-6}$ portant 1 à 4 substituants hydroxy, ou $YR^5$ où Y représente un atome de soufre ou d'oxygène et $R^5$ représente un groupe alkyle en $C_{1-6}$ substitué ou non, un groupe aryle substitué ou non, un groupe hétéroaryle substitué ou non, ou un résidu d'éther cyclique substitué ou non ;
$R^2$ et $R^3$ représentent chacun, indépendamment, un atome d'hydrogène, un groupe alkyle en $C_{1-6}$, ou un groupe aralkyle substitué ou non, ou bien $R^2$ et $R^3$ représentent ensemble un groupe méthylène ou éthlène, substitué ou non ;
$R^4$ représente un atome d'hydrogène ou d'halogène ou un groupe hydroxy, alkyle en $C_{1-6}$, aralkyle substitué ou non, alcoxy en $C_{1-6}$, ou aralcoxy substitué ou non ;

et X représente un groupe indolyle substitué ou non ;
un groupe alkyle en $C_{1-6}$ substitué portant 1 à 4 substituants, identiques ou différents et choisis dans l'ensemble formé par les atomes d'halogène, les groupes vinyle, hydroxy, alcoxy en $C_{1-6}$, aryloxy, amino, (alkyle en $C_{1-6}$) amino, di(alkyle en $C_{1-6}$)amino, (alcanoyle en $C_{2-7}$)amino, (alcoxy en $C_{1-6}$)carbonylamino, nitro, carboxy, alcanoyle en $C_{2-7}$, (alcoxy en $C_{1-6}$)-carbonyle et tri(alkyle en $C_{1-6}$)silyle, et les résidus d'éther cyclique ;
un groupe aryle substitué, hétéroaryle substitué, aralkyle substitué ou aralcoxy substitué, ainsi qu'un résidu d'éther cyclique substitué, portant dans chaque cas 1 à 4 substituants, identiques ou différents et choisis dans l'ensemble formé par les atomes d'halogène, les groupes alkyle en $C_{1-6}$, vinyle, hydroxy, hydroxyméthyle, alcoxy en $C_{1-6}$, aryloxy, amino, (alkyle en $C_{1-6}$)amino, di(alkyle en $C_{1-6}$)amino, (alcanoyle en $C_{2-7}$)amino, (alcoxy en $C_{1-6}$)carbonylamino, nitro, carboxy, alcanoyle en $C_{2-7}$, (alcoxy en $C_{1-6}$)-carbonyle, tri(alkyle en $C_{1-6}$) silyle et aralkyle, et les résidus d'éther cyclique ;
un groupe éthylène substitué ou méthylène substitué portant dans chaque cas 1 à 3 substituants alkyle en $C_{1-6}$, identiques ou différents ;
un groupe indolyle substitué pouvant porter, sur l'atome d'azote en position 1, un substituant choisi dans l'ensemble formé par les groupes alkyle en $C_{1-6}$, alcanoyle en $C_{2-7}$, (alcoxy en $C_{1-6}$)carbonyle et aralkyle, et sur les atomes de carbone en positions 2 à 7, un ou des substituants choisis dans l'ensemble formé par les atomes d'halogène et les groupes alkyle en $C_{1-6}$, alcoxy en $C_{1-6}$, amino, (alkyle en $C_{1-6}$)amino, di(alkyle en $C_{1-6}$)amino, (alcanoyle en $C_{2-7}$)amino, (alcoxy en $C_{1-6}$)carbonylamino, nitro, carboxy, alcanoyle en $C_{2-7}$, (alcoxy en $C_{1-6}$)-carbonyle et aralkyle ;
un groupe aryle et le fragment aryle d'un groupe aryloxy étant un groupe phényle ou naphtyle ;
un groupe hétéroaryle étant un groupe pyridyle, pyrazinyle, pyrimidinyle, pyridazinyle, quinolyle, isoquinolyle, phtalazinyle, quinazolinyle, quinoxalinyle, naphtyridinyle, pyrrolyle, pyrazolyle, imidazolyle, triazolyle, tétrazolyle, thiényle, furyle, thiazolyle, oxazolyle, indolyle, indazolyle, benzimidazolyle, benzotriazolyle ou purinyle ;
un résidu d'éther cyclique étant un résidu d'éther cyclique comportant 2 à 6 atomes de carbone ;
et un groupe aralkyle et le fragment aralkyle d'un groupe aralcoxy étant un groupe aralkyle comportant 7 à 15 atomes de carbone ;

ou sel admissible en pharmacie d'un tel composé.

2. Dérivé de propénone ou sel admissible en pharmacie d'un tel composé, conforme à la revendication 1, dans lequel $R^1$ représente $YR^5$ où Y et $R^5$ ont les significations indiquées dans la revendication 1.

3. Dérivé de propénone ou sel admissible en pharmacie d'un tel composé, conforme à la revendication 1, dans lequel $R^1$ représente un groupe alkyle en $C_{1-6}$ portant 1 à 4 substituants hydroxy.

4. Dérivé de propénone ou sel admissible en pharmacie d'un tel composé, conforme à l'une des revendications 1 à 3, dans lequel X représente un groupe indol-3-yle substitué ou non.

5. Composition pharmaceutique comprenant un dérivé de propénone ou un sel admissible en pharmacie d'un tel composé, conforme à l'une des revendications 1 à 4.

6. Emploi d'un dérivé de propénone ou d'un sel admissible en pharmacie d'un tel composé, conforme à l'une des revendications 1 à 4, en vue de la préparation d'une composition pharmaceutique destinée au traitement de tumeurs.

7. Emploi d'un dérivé de propénone ou d'un sel admissible en pharmacie d'un tel composé, conforme à l'une des revendications 1 à 4, en vue de la préparation d'une composition pharmaceutique dotée d'une activité immunosuppressive.